# EUROPEAN PATENT SPECIFICATION

(11) **EP 1 007 024 B1**
(45) Date of publication and mention of the grant of the patent: **23.07.2003**
(21) Application number: 98923022.2
(22) Date of filing: 16.06.1998
(51) Int. Cl.: A61K 31/135, A61K 9/28, A61P 25/00

(54) **DELAYED-RELEASE DOSAGE FORMS OF SERTRALINE**
VERABREICHUNGSFORM FÜR SERTRALIN MIT VERZÖGERTER WIRKSTOFFFREIGABE
FORMES POSOLOGIQUES DE SERTRALINE, A ACTION RETARDEE

(30) Priority: 01.07.1997 US 51499 P
(43) Date of publication of application: 14.06.2000
(73) Proprietor: Pfizer Products Inc., Groton, Connecticut 06340 (US)
(72) Inventor: CURATOLO, William, John, Niantic, CT 06357 (US); FRIEDMAN, Hylar, Lewis, Brattleboro, VT 05301 (US)
(74) Representative: Ruddock, Keith Stephen
(86) International application number: IB9800937
(87) International publication number: WO99001122

(56) References cited:
- EP-A- 0 080 341
- EP-A- 0 259 113
- EP-A- 0 357 369
- WO-A-90/14077

## Description

### Field of the Invention

This invention relates to a delayed-release dosage form of sertraline having a shorter time to reaching peak plasma levels after oral dosing. It also relates to dosage forms having an improved side effect profile and to the manufacture of a medicament for treating psychiatric and other illnesses comprising administering sertraline in such a delayed-release dosage form to a mammal, including a human patient, in need of such treatment.

### Background of the Invention

Sertraline is a selective serotonin reuptake inhibitor (SSRI), which is useful as an antidepressant and anorectic agent, and in the treatment of obsessive-compulsive disorder, post-traumatic stress disorder, anxiety-related disorders, and panic. Sertraline is also useful for the treatment of premature ejaculation, chemical dependencies, premenstrual dysphoric disorder and obesity.

Sertraline is most commonly prescribed for therapy of depressive illness in the general dose range 50-200 mg/day. Sertraline has an elimination half-life of 23 hr, and is dosed once daily.

Patients are generally initiated on sertraline for depression at a dose of 50 mg/day. Patients who do not respond at the 50 mg dose are given higher doses. Initiation at doses greater than 50 mg is generally avoided, when possible, because side effects such as nausea, diarrhea and regurgitation, are generally believed to be more severe at higher doses. If necessary to achieve efficacy, higher doses may be reached by titration up from lower doses. Improved sertraline dosage forms which exhibited a lower incidence and/or severity of side effects would be advantageous because (1) patient comfort would be improved, and (2) dosing could be initiated at doses higher than 50 mg without the need for dose titration. Initiation at higher starting doses may, in turn, be useful by effecting a shorter onset of antidepressive action. Thus, such an improved sertraline dosage form which permitted oral dosing of high doses of sertraline (e.g., 200 mg) with relatively reduced side effects would permit wider therapeutic application of sertraline therapy, and would accordingly provide a significant improvement in dosing compliance and convenience. Ukewise, an improved dosage form which lowered the incidence of side-effects at lower doses would also be of significant value.

With respect to known, immediate-release dosage forms of sertraline, upon oral administration of such dosage forms, Tₘₐₓ, the time at which a maximal plasma sertraline concentration is achieved, is approximately 6-7 hours. Generally speaking, this is a long Tₘₐₓ. It has now been determined that oral dosage forms which retain their contained sertraline until the dosage form exits the stomach and enters the small intestine are capable of delivering sertraline to the systemic circulation faster, and with a shorter Tₘₐₓ, than conventional dosage forms which immediately start to disintegrate and dissolve following ingestion. The fact that delaying dissolution of a drug in the gastrointestinal tract results in faster appearance of the drug in the bloodstream is counterintuitive and surprising.

Delivery of sertraline in a dosage form which minimizes gastric exposure can also have other unexpected benefits. It is herein demonstrated (see the examples) that certain side effects of sertraline, namely nausea, regurgitation, and diarrhea, are partially or primarily mediated by direct contact of sertraline with the upper gastrointestinal tract, primarily the stomach, rather than mediated systemically, that is via exposure of sertraline to the bloodstream after absorption. Prior to the human dinical studies disclosed herein, the locally mediated nature of these three sertraline side effects was not known. It is noted that such side effects are not universally locally mediated for all drugs which elicit them. For example, cancer chemotherapeutants which are dosed by injection can elicit these same side effects.

### Summary of the Invention

This invention provides an oral delayed release dosage form of sertraline which decreases Tₘₐₓ relative to the Tₘₐₓ exhibited by currently known immediate-release sertraline tablet dosage forms which deliver an equivalent bolus dose. Relative to immediate release dosage forms, this invention can also decrease the incidence and/or severity of gastrointestinal and/or other side effects. The delayed release period is followed by immediate release, as described below, and such dosage forms are sometimes referred to herein as "delayed release plus immediate release" forms for convenience. A delayed release plus immediate release dosage form is within the scope of the invention if it decreases Tₘₐₓ or if it reduces any of the side effects previously noted.

The dosage form can operate by being sensitive to its use environment such that it delays releasing sertraline until after it has passed into the small intestine. This type of delayed release dosage form releases in a manner which is dependent on position along the gastrointestinal (GI) tract, is independent of time, and is herein referred to as a "spatial" dosage form, or as exhibiting "spatially delayed release". After the dosage form has entered the small intestine, it releases its remaining sertraline in immediate fashion, "immediate release" meaning that no component or means is implemented in the dosage form which would deliberately retard or slow down release once the delay period has ended: In general, the dosage form should release at least 70% of the sertraline remaining therein within 1.5 hours, preferably within one hour, after passing into the small intestine. Examples of spatially delayed dosage forms are (1) pH-triggered dosage forms which delay release of sertraline until they enter the environment of the small intestine, which is above pH 6.0, and (2) small intestinal enzyme-triggered dosage forms which delay release of sertraline until a coating on the dosage form is altered by interaction with lipases, esterases, or proteases in the small intestinal lumen, as appropriate. Spatially-delayed dosage forms of this invention generally commence immediate release of sertraline within approximately 30 minutes, preferably within 15 minutes, after passing out of the stomach into the small intestine.

Thus, in one aspect this invention provides a spatially delayed-release oral dosage form suitable for oral administration to a mammal, comprising sertraline or a pharmaceutically acceptable salt thereof and a pharmaceutically acceptable carrier,
which dosage form, following ingestion by said mammal, releases not more than 10% of the sertraline contained therein into said mammal's stomach,
and which effects immediate release of the remaining sertraline contained therein after having passed into said mammal's small intestine.

Extents of sertraline release into the stomach lower than the 10% extent disclosed above are also within the scope of the invention and may produce even shorter Tₘₐₓ's and/or better side effect profiles. Thus a dosage form which releases 5% or less of its contained sertraline into a mammal's stomach prior to effecting immediate release once having entered the small intestine represents a release profile within the scope of the invention and may be even more efficacious for shortening Tₘₐₓ and/or ameliorating side effects. It is preferred that the dosage form release an even smaller amount of sertraline into the stomach, more preferably not more than 3% of the sertraline contained therein. It is most preferred that the dosage form release essentially no sertraline into the stomach.

As mentioned, the spatially delayed form can be enzyme-triggered or pH triggered. Both of these dosage form embodiments can be dissolution tested in *in vitro* tests which offer a good approximation of *in vivo* behavior, thereby determining whether they fall within the scope of the invention. Thus in another aspect, a pH-triggered *in vitro* test, this invention provides a pH-triggered delayed release dosage form suitable for oral adminstration to a mammal, comprising (1) an immediate-release core comprising sertraline or a pharmaceutically acceptable salt thereof and a pharmaceutically acceptable carrier, and (2) a pH-sensitive coating surrounding said core,
which dosage form, when dissolution tested *in vitro*,
releases not more than 10% of its incorporated sertraline in 2 hours in 750 ml of 0.1 N HCl,
and which, following said 2 hours, effects immediate release of its remaining sertraline in a liter of 0.05 M sodium phosphate buffer, pH 6.8, containing 1% polysorbate 80. Again, "immediate release" as used herein generally means at least 70% release within 1.5 hours, preferably within 1 hour.

In another aspect, an enzyme-triggered *in vitro* test, this invention provides an enzyme-triggered delayed release dosage form suitable for oral adminstration to a mammal, comprising (1) an immediate-release core comprising sertraline or a pharmaceutically acceptable salt thereof and a pharmaceutically acceptable carrier, and (2) an enzymatically degradable coating surrounding said core,
which dosage form, when dissolution tested *in vitro*,
releases not more than 10% of its incorporated sertraline in 2 hours in 750 ml of 0.1 N HCI,
and which, following said 2 hours, effects immediate release of its remaining sertraline in a liter of 0.05 M sodium phosphate buffer, pH 6.8, containing 1% polysorbate 80, in the presence of an enzyme suitable for enzymatically degrading said coating. The actual enzyme employed in the test will depend on which duodenal or small intestinal enzyme the enzyme-triggered coating is susceptible to.

It is noted that the *in vitro* acid medium simulates the environment of the stomach. The buffer simulates the environment of the small intestine.

In addition to the spatial delayed release dosage forms discussed above, a dosage form according to the invention can also operate by delaying the release of sertraline for a set period of time, decreasing the exposure of the stomach to sertraline. This type of dosage form is referred to herein as a "temporal" dosage form, or as exhibiting "temporally delayed release" or similar language. A temporal delay is a delay occurring after the dosage form is ingested, which delay is not related to the spatial location of the dosage form in the gastrointestinal tract. Temporally delayed dosage forms may be considered to be triggered by the presence of water, and possess a means for delaying release of sertraline for a specific time period after the dosage form enters an aqueous environment.

Thus, in another aspect this invention provides a temporally delayed dosage form suitable for oral adminstration to a mammal, comprising (1) an immediate-release core comprising sertraline or a pharmaceutically acceptable salt thereof and a pharmaceutically acceptable carrier and (2) a coating surrounding said core,
which dosage form, following ingestion by said mammal,
releases substantially no sertraline during a first period of about 10 minutes,
releases not more than 10% of the sertraline contained therein during a second period lasting up to 2 hours following said first period,
and then effects immediate release of the remaining sertraline contained therein. The dosage form works generally by (1) the coating dissolving, disintegrating or becoming otherwise more permeable to sertraline in an aqueous environment within a pre-set period of time (i.e., the delay period), thereafter releasing sertraline in immediate fashion or (2) the combination of the core and coating (e.g., a semipermeable coating) being physically disrupted by imbibing water through the coating until the core and coating burst, thereby immediately releasing sertraline.

It is noted that the first period of "about 10 minutes" accounts for the natural lag time or induction period following swallowing (i.e. ingestion) characteristic of most if not all solid dosage forms, including pure immediate release forms, during which the dosage form is wetted and/or hydrated. The period is of course variable, "about" meaning on the order of 2-20 minutes. The second period accounts for the delay period which has actually been purposely engineered into the dosage form. For spatially delayed dosage forms, the first period lag time is subsumed under the delay period during which not more than 10% of the contained sertraline is released.

A temporally delayed dosage form releasing "substantially no sertraline" during a first period means that the dosage form releases as dose to 0% sertraline as possible, although "substantially" certainly allows for de minimis amounts of release, preferably 1% or less. It is further noted that the aforementioned second period can last "up to 2 hours", which means the period can be less than two hours.

Temporally delayed dosage forms can also be dissolution tested in an *in* vitro test which mimics or approximates *in vivo* behavior, thereby determining whether they fall within the scope of the invention. Thus in another aspect, a temporal *in vitro* test, this invention provides a temporally delayed dosage form suitable for administration to a mammal, comprising sertraline or a pharmaceutically acceptable salt thereof and a pharmaceutically acceptable carrier,
which dosage form, when dissolution tested in vitro in a USP-2 apparatus containing 900 ml of acetic acid/acetate buffer, pH 4.0, which is 0.075 M in NaCI,
releases substantially no sertraline during a first period of about 10 minutes,
releases not more than 10% of the sertraline contained therein during a second period lasting up to 2 hours following said first period,
and which then effects immediate release of the remaining sertraline contained therein following said second period.

As mentioned, the invention is surprising in that the dosage forms herein, even though they delay releasing sertraline in the GI tract, shorten Tₘₐₓ, the time it takes for sertraline to reach its maximum value in the blood. This shortening of Tₘₐₓ for sertraline is novel and is provided as a further feature of the invention. Thus this invention further provides a delayed-release dosage form suitable for oral administration to a mammal, comprising sertraline or a pharmaceutically acceptable salt thereof and a pharmaceutically acceptable carrier, said dosage form exhibiting, *in vivo*, a plasmic Tₘₐₓ which is shorter than Tₘₐₓ determined after ingestion of an equivalent amount of sertraline in an immediate release dosage form (i.e. one that has no delay period engineered or implemented therein). Preferred delayed release dosage forms exhibit, relative to an immediate release dosage form containing an equal amount of sertraline, a Tₘₐₓ that is shorter by at least 1/2 hr, preferably at least 1 hr.

It is well known that the retention time of a dosage form in the stomach depends upon whether the subject has eaten. Certain dosage forms, e.g., non-disintegrating tablets, will remain in the stomach until the meal has substantially passed on into the duodenum, said gastric retention period lasting as long as three hours. Multiparticulate dosage forms will also spend a longer time in the fed stomach than in the fasted stomach, although, in this case, the increased duration is reflected in a longer half-time for gastric emptying of these small multiparticulates, which may range in diameter from about 50 microns to about 0.3 cm. Thus, with respect to the property of decreasing Tₘₐₓ, it is preferred that the dosage forms of this invention be ingested when the subject is in the fasted state, e.g., more than 1 hour before or more than 2 hours after a meal. With respect to the Tₘₐₓ-shortening property, dosage forms of this invention are variably effective in fed subjects, depending on the relative timing of dosing and meal ingestion, and upon the caloric content of the meal. With respect to the side effect amelioration property, spatially delayed dosage forms exhibit no fed/fasted preference. With respect to the side effect amelioration property, temporally delayed sertraline dosage forms are preferably dosed in the fasted state and have more variable effectiveness in the fed state.

The amount of sertraline contained within a delayed release dosage form is at least 10 mg, and can be as high as 300 mg or more. The amount contained in the dosage form is preferably 10 mg to 250 mg. The dosage form can be unitary, or divided e.g., constituted by two or more units (such as capsules or tablets which, taken together, constitute the dosage form) which are taken at or about the same time.

Sertraline can be employed in the dosage forms of this invention in the form of the free base or its pharmaceutically acceptable salts such as the hydrochloride, aspartate, acetate, or lactate, and also in anhydrous as well as hydrated forms. All such forms can be used within the scope of this invention.

The salts can generally be made by combining sertraline free base with a corresponding stoichiometric amount of acid (i.e. aspartic, acetic or lactic acid), as further described in commonly assigned Pfizer Docket No. 9337AJTJ, filed as a PCT application designating the United States, and herein incorporated by reference in its entirety. The sertraline employed is preferably the free base, hydrochloride, aspartate, acetate, or lactate. Reference to "sertraline" in terms of therapeutic amounts or in release rates in the claims is to active sertraline, abbreviated herein as "mgA", i.e., the non-salt, non-hydrated free base having a molecular weight of 306.2. Amounts in mgA can easily be converted to an equivalent weight for any salt.

The dosage forms which constitute the subject matter of the invention are, as mentioned, delayed release formulations. The dosage form can be in the form of a tablet, a capsule, a multiparticulate form, or a unit dose packet (sometimes referred to in the art as a "sachet"). Also induded are combination dosage forms, for example those comprising one or more delayed release tablets contained within a capsule shell such as a gelatin capsule shell.

The term "tablet" is intended to embrace compressed tablets which are coated with materials which elicit the desired delayed release effect. Tablet dosage forms may be "unitary", in which the entire dose is incorporated in a single tablet, or may be "multiple", in which the dose is incorporated in more than one tablet, which may be ingested at about the same time, or may be incorporated into a capsule which dissolves after ingestion, releasing multiple tablets. Tablets comprise preferred dosage forms of this invention because of the well developed art in the production and coating of tablets.

The term "capsule" is intended to embrace capsules in which the body of the capsule disintegrates after ingestion to release particulate contents which exhibit the desired delayed-release behavior, and also capsules for which the body of the capsule provides the delayed release mechanism. Also included are hard or soft gelatin capsules which contain solutions or suspensions of sertraline. Delayed release encapsulated solution dosage forms of sertraline are preferred because of their capacity to directly provide sertraline in solution, maximizing the Tₘₐₓ-reducing properties of the dosage form.

The term "multiparticulate" is intended to embrace a dosage form comprising a multiplicity of partides whose totality represents the intended therapeutically useful dose of sertraline. The particles generally have a diameter from about 50 microns to about 0.3 cm, with a preferred range of 100 µM to 2 mm. Multiparticulates represent a preferred embodiment because they are amenable to use in scaling dosage forms according to the weight of an individual animal (e.g., a dog) by simply scaling the number of particles in the dosage form to conform with the animal's weight. Multiparticulates are also preferred because they undergo more reproducible gastric emptying than do larger unitary dosage forms (e.g. tablets), particularly with respect to differences in gastric emptying in the fed and fasted states. Diameters in the range of 0.4 to 2 mm are preferred as beads for use as capsule fill. Diameters in the range of 0.2 to 1 mm are preferred for compression into tablets. Diameters in the range of 0.1 to 0.8 mm are preferred for use as powders for making powders for oral suspension or unit dose packs ("sachets").

Multiparticulate, bead, or other partide dosage forms may be multiply loaded into a gelatin capsule, or may be compressed into a tablet.

In a further aspect, this invention provides the manufacture of a medicament for treating a psychiatric or other illness, comprising administering to a mammal in need of such treatment, including a human patient, a therapeutically effective amount of sertratine in a delayed-release oral dosage form which releases the sertraline according to the release criteria described above. Such psychiatric illnesses include those known in the art as being treatable with sertraline, including those mentioned above. Obesity, premenstrual dysphoric disorder, chemical dependencies and premature ejaculation are also treatable with the delayed release plus immediate release dosage forms of this invention.

It is an object of this invention to provide a dosage form of sertraline which has a shorter Tₘₐₓ than conventional sertraline dosage forms, thus permitting faster appearance of sertraline in the bloodstream, and a potentially faster therapeutic effect. A faster therapeutic effect is of particular importance in acute indications such as the amelioration of panic or premature ejaculation. It is a further object of this invention to decrease the incidence and severity of sertraline-induced GI side effects. This is important at all doses, and particularly at high doses, for example 200 mg and up, at which the incidence of gastrointestinal side effects can be relatively high. This object is effected by minimizing the extent and duration of exposure of the stomach to sertraline, thereby reducing the overall incidence and severity of sertratine-induced nausea, regurgitation, or diarrhea.

Dosing sertraline orally in conventional immediate release tablets (Zoloft®, registered trademark of Pfizer Inc.) results in relatively extensive exposure of drug to the stomach. It is accordingly a further object of this invention to provide dosage forms which deliver therapeutically useful doses of sertraline, while reducing localized exposure of sertraline to the upper GI tract, especially the stomach, and reducing Tₘₐₓ and hastening therapeutic sertraline exposure in the systemic circulation, with the added benefit of decreased nausea, regurgitation, or diarrhea.

### Brief Description of the Figure

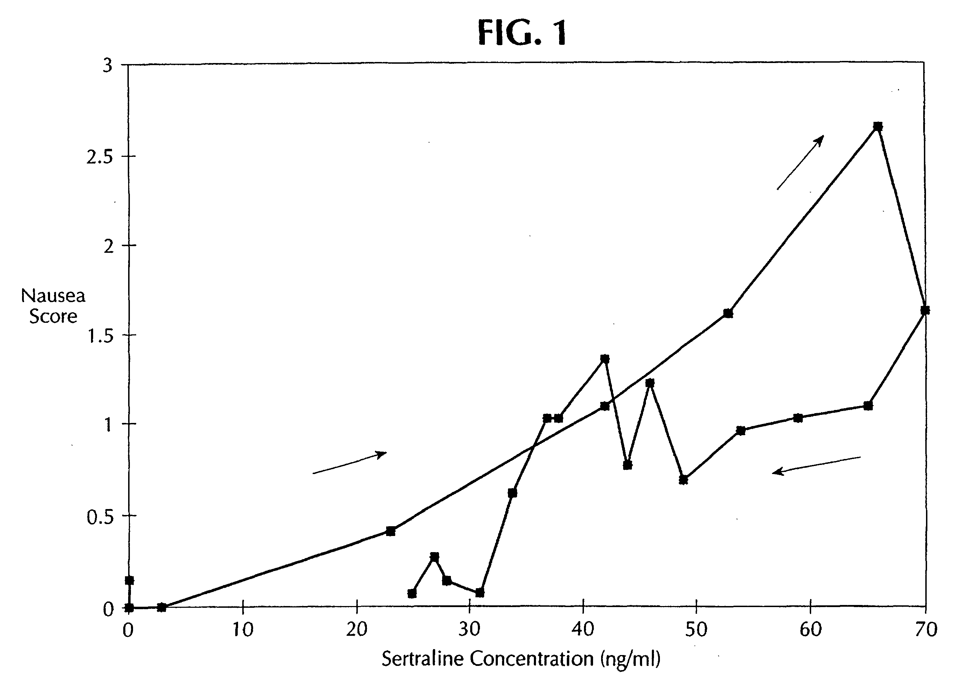

Figure 1 is a PK/PD plot which presents the relationship between plasma sertraline concentration and average self-reported visual analogue scores for a nausea study presented in the Examples.

### Detailed Discussion

In principle, the invention can be implemented by taking an immediate release core comprising sertraline and a pharmaceutically acceptable carrier and coating it with a (preferably all-covering) coating which provides the desired delayed release characteristics, either by a spatial or temporal mechanism. Thus any immediate release sertraline dosage form can be used as a core which is in tum coated with a desired delayed-release coating, and such dosage forms constitute preferred embodiments within the scope of this invention.

### Spatially Delayed pH-triggered Dosage Forms

A first spatially-delayed release embodiment according to the invention is a "pH-dependent coated tablet", which comprises an immediate-release tablet or tablet core coated with a material comprising a polymer that is substantially impermeable to sertraline at the pH of the stomach, but which becomes permeable to sertraline at the pH of the small intestine. "Substantially impermeable" in relation to spatially delayed dosage forms allows for very small amounts of sertraline to be released through the coating, so long as not more than 10% of the sertraline contained in the dosage form is released in the stomach. Such polymers become permeable by virtue of dissolving or disintegrating or otherwise being disrupted so that sertraline can freely pass through. The tablet or tablet core can comprise further excipients such as disintegrants, lubricants, fillers, and/or other conventional formulation ingredients. All such ingredients and/or excipients, regardless of the particular dosage form, are referred to herein collectively as the pharmaceutically acceptable "carrier". The core is coated with a material, preferably a polymer, which is substantially insoluble and impermeable at the pH of the stomach, but which is more permeable at the pH of the small intestine. Preferably, the coating polymer is substantially insoluble and impermeable at pH <5.0, and water-soluble or water-disintegrable at pH>5.0. Mixtures of a pH-sensitive polymer with a water-insoluble polymer may also be employed. Tablets are coated with an amount of polymer comprising from 3% to 70% of the weight of the sertraline-containing tablet core. Preferred tablets are coated with an amount of polymer comprising 5% to 50% of the weight of the sertraline-containing tablet core.

pH-sensitive polymers which are relatively insoluble and impermeable at the pH of the stomach, but which are more soluble or disintegrabile or permeable at the pH of the small intestine and colon include polyacrylamides, phthalate derivatives such as acid phthalates of carbohydrates, amylose acetate phthalate, cellulose acetate phthalate, other cellulose ester phthalates, cellulose ether phthalates, hydroxypropylcellulose phthalate, hydroxypropylethylcellulose phthalate, hydroxypropylmethylcellulose phthalate, methylcellulose phthalate, polyvinyl acetate phthalate, polyvinyl acetate hydrogen phthalate, sodium cellulose acetate phthalate, starch acid phthalate, cellulose acetate trimellitate, styrene-maleic acid dibutyl phthalate copolymer, styrene-maleic acid polyvinylacetate phthalate copolymer, styrene and maleic acid copolymers, polyacrylic add derivatives such as acrylic acid and acrylic ester copolymers, polymethacrylic acid and esters thereof, poly acrylic methacrylic add copolymers, shellac, and vinyl acetate and crotonic add copolymers.

Preferred pH-sensitive polymers include shellac, phthalate derivatives, particularly cellulose acetate phthalate, polyvinylacetate phthalate, and hydroxypropylmethylcellulose phthalate; cellulose acetate trimellitate; polyacrylic acid derivatives, particularly copolymers comprising acrylic acid and at least one acrylic acid ester, polymethyl methacrylate blended with acrylic add and acrylic ester copolymers; and vinyl acetate and crotonic add copolymers.

A particularly preferred group of pH-sensitive polymers includes cellulose acetate phthalate, polyvinylacetate phthalate, hydroxypropylmethylcellulose phthalate, anionic acrylic copolymers of methacrylic acid and methylmethacrylate, and copolymers comprising acrylic acid and at least one acrylic add ester.

Cellulose acetate phthalate (CAP) may be applied to sertraline tablets to provide delayed release of sertraline until the sertraline-containing tablet has exited the stomach. The CAP coating solution may also contain one or more plasticizers, such as diethyl phthalate, polyethyleneglycol-400, triacetin, triacetin citrate, propylene glycol, and others as known in the art. Preferred plasticizers are diethyl phthalate and triacetin. The CAP coating formulation may also contain one or more emulsifiers, such as polysorbate-80.

Anionic acrylic copolymers of methacrylic acid and methylmethacrylate are also particularly useful coating materials for delaying the release of sertraline from sertraline-containing tablets until the tablets have moved to a position in the GI tract which is distal to the stomach. Copolymers of this type are available from RöhmPharma Corp, under the trademarks Eudragit®-L and Eudragit®-S. Eudragit®-L and Eudragit®-S are anionic copolymers of methacrylic add and methylmethacrylate. The ratio of free carboxyl groups to the esters is approximately 1:1 in Eudragit®-L and approximately 1:2 in Eudragit®-S. Mixtures of Eudragit®-L and Eudragit®-S may also be used. For coating of sertraline-containing tablets, these acrylic coating polymers can be dissolved in an organic solvent or mixture of organic solvents or suspended in aqueous media. Useful solvents for this purpose are acetone, isopropyl alcohol, and methylene chloride. It is generally advisable to include 5-20% plasticizer in coating formulations of acrylic copolymers. Useful plasticizers include polyethylene glycols, propylene glycols, diethyl phthalate, dibutyl phthalate, castor oil, and triacetin. Eudragit®-L is preferred because it dissolves relatively quickly at intestinal pH.

The coating, as noted above, may comprise from 3% to 70% of the weight of the uncoated tablet core. Preferably, the coating comprises from 5% to 50%, more preferably 5% to 40% of the weight of the tablet core.

In a further embodiment of a spatially-delayed sertraline dosage form, a "pH-dependent coated bead", beads 0.4 to 2.0 mm in diameter comprising sertraline plus carrier are coated with one or more of the aforementioned pH-sensive polymers. The coated beads may be placed in a capsule or may be compressed into a tablet, with care taken to avoid damaging the polymeric coat on individual beads during tablet compression. Preferred coated beads are those which exhibit essentially no release (i.e., less than 10 %) of sertraline from the dosage form, as previously discussed, until the beads have exited the stomach, thus assuring that minimal sertraline is released in the stomach. The coating may comprise from 5% to 200% of the weight of the uncoated bead core. Preferably, the coating comprises from 10% to 100% of the weight of the bead core.

In a further embodiment of a multiparticulate spatially-delayed sertraline dosage form, a "pH-dependent coated particle", the dosage form comprises small sertraline plus carrier particles from 0.1 to 0.4 mm in diameter. The particles are coated with one or more of the aforementioned pH-sensitive polymers. The coated particles may be used to make unit dose packs or may be placed in a capsule or may be compressed into a tablet, with care taken to avoid damaging the polymeric coat on individual particles during tablet compression. Preferred coated particles are those which exhibit essentially no release of sertraline from the dosage form (i.e. less than 10%) until the particles have exited the stomach, thus assuring that minimal sertraline is released in the stomach. Mixtures of a pH-sensitive polymer with a water-insoluble polymer are also included. Preferred sertraline-containing particles are coated with an amount of polymer comprising 15% to 200% of the weight of the uncoated sertraline-containing partide core.

Mixtures of a pH-sensitive polymer with a water-insoluble polymer are also included. Sertraline-containing tablets and particles and beads may be coated with mixtures of polymers whose solubilities vary at different pH's. For example, preferred coatings comprise Eudragit®-L, or from 9:1 to 1:4 Eudragit®-L/Eudragit®-S.

A further embodiment of a spatially-delayed sertraline dosage form constitutes a modification of the pH-dependent coated tablet, pH-dependent coated bead, and pH-dependent coated particle embodiments. The sertraline-containing core tablet, bead, or particle is first coated with a barrier coat, and then is coated with the pH-dependent coat The function of the barrier coat is to separate sertraline from the pH-dependent coat. Since sertraline is a base, hydration of the sertraline in the core can serve to raise the pH in the microenvironment of the pH-dependent coating, thus prematurely initiating the permeabilization or dissolution of the pH-dependent coating, resulting in premature release of some or all of the sertraline dose in the stomach. A barrier coat prevents such premature release. Suitable barrier coatings are composed of water-soluble materials such as sugars such as sucrose, or water-soluble polymers such as hydroxypropyl cellulose, hydroxypropyl methylcellulose, and the like. Hydroxypropyl cellulose and hydroxypropylmethylcellulose and Polyvinylpyrrolidone are preferred. The barrier coat may comprise from 1% to 20%, preferably from 2% to 15%, of the weight of the uncoated sertraline-containing tablet, bead or particle core.

In a further embodiment of a spatially-delayed sertraline dosage form, a solution or suspension or powder of sertraline in a solvent is encapsulated in a water soluble capsule, such as a hard or soft gelatin capsule as known in the art, and the capsule is coated with a pH-dependent polymer as described above for "pH-dependent coated tablets". In the preparation of sertraline solutions for encapsulation, solvents such as trigtyceride oils and glycols may be used. Useful and preferred sertraline solvents are disclosed in commonly assigned co-pending provisional application [Pfizer Docket 9838JTJ], filed on even date herewith and herein incorporated by reference. Useful and preferred solvents are also enumerated below.

Preferred solvents are water-immiscible solvents including water-immiscible oils, including triglyceride vegetable oils such as safflower oil, sesame oil, olive oil, com oil, castor oil, coconut oil, cottonseed oil, soybean oil, and the like. Also included are synthetic and semisynthetic medium chain triglyceride oils such as those sold under the tradename Miglyol® (HulsAmerica, Piscataway, New Jersey) or Captex® (Abitec Corp., Columbus, Ohio). Examples are triglycerides of caprylic/capric acids (Miglyol®-810, Miglyol®-812, Captex®-300, Gaptex®-355), and triglycerides of caprylic/capric/linoleic acids (Miglyol-818). Also included are long chain triglyceride oils such as triolein, and other mixed chain triglycerides which are liquid at room temperature.

Water-immiscible solvents also indude monoglycerides and diglycerides such as those sold under the trademarks Capmul® (ABITEC, Columbus, Ohio) and Imwitor® (HulsAmerica, Piscataway, New Jersey). Examples are monoolein (Capmul®-GMO), mono and diglycerides of octanoic and decanoic acids (Imwitor®-742, Capmul®-MCM), and monooctanoin (Imwitor®-308), and the like.

Preferred oils are liquid at room temperature. Preferred mono-, di-, and triglycerides are those with an average acyl chain length of C4-C18.

Useful vehicles further include various liquid esters of short chain alcohols, such as the propylene glycol ester of caprylic/capric acids (Miglyol®-840, Captex®-200). Fatty acids which are liquid at room or body temperature, such as caprylic acid, capric acid, lauric acid, oleic acid, or linoleic acid are also useful.

Further useful vehicles include semisolid vehicles such as those sold under the registered trademark Gelucire®. Examples are PEG-32-glyceryl-laurate (Gelucire® 44/14), and glycerol esters of fatty acids (Gelucire® 33/01).

Further useful vehicles also include surfactants and emulsifiers which have the capacity to dissolve sertraline. These surfactants and emulsffiers form micelles when they are mixed with aqueous media. Examples are polysorbate-80, nonylphenoxypolyoxyethylenes, dioctyl sodium sulfosuccinate, PEG-6 glyceryl monooleate (Labrafil® M-1944-CS), PEG-6 glyceryl linoleate (Labrafil® M-2125-CS), and the like.

Preferred vehicles are those which can dissolve sertraline or one of its pharmaceutically acceptable salts at a concentration of 16.7 mgA/ml or greater. Certain encapsulation vehicles have a higher capacity than others for maintaining sertraline in solution after the formulation has mixed with simulated gastrointestinal contents. More preferred vehicles are those which inhibit precipitation of sertraline in the presence of either 0.1N HCl or phosphate buffered saline, pH 5.8. These encapsulation vehicles are more preferred because they minimize precipitation or gelling of sertraline in the use environment, i.e. the gastrointestinal lumen, thus maximizing the speed with which sertraline can appear in the bloodstream after dosing. Even if these preferred vehicles do not completely or almost completely prevent the precipitation of sertraline when mixed with chloride-containing model physiological fluids, any effect on sertraline predpitation rate is advantageous. In vivo, the intestinal wall has a high capacity for rapidly absorbing sertraline, revealed by a high absorption rate constant (ARC). Any formulation which helps keep sertraline in solution, even temporarily, will be useful because precipitation and absorption compete for the available soluble sertraline.

More preferred vehicles, according to this criterion, are vegetable oils such as safflower oil and olive oil; medium chain triglycerides such as caprylic/capric triglycerides; mono- and di-glycerides induding medium chain mono- and di-glycerides; acylated polyols such as propylene glycol dicaprylate/caprate; fatty acids such as oleic acid; and surfactants such as polysorbate-80.

Most preferred vehicles are those which inhibit sertraline precipitation in 0.1N HCl and in phosphate buffered saline, pH 5.8. These include medium chain triglycerides such as caprylic/capric triglycerides; mono- and di-glycerides including medium chain mono- and di-glycerides; acylated polyols such as propylene glycol dicaprylate/caprate; fatty acids such as oleic acid; and surfactants such as polysorbate-80. Most preferred vehicles have the capacity to solubilize sertraline hydrochloride in the use environment, thus minimizing the precipitation of this salt in chloride-containing physiological solutions, regardless of whether sertraline has been originally dosed as free base, hydrochloride salt, or other pharmaceutically acceptable salt. Most preferred vehicles exhibit a sertraline hydrochloride salt solubility greater than 0.3 mgA/ml (to inhibit sertraline predpitation in physiological fluids), in addition to exhibiting a sertraline solubility greater than 16.7 mgA/ml for any form of sertraline (to permit dosing 10 mgA or more in a 0.8 ml gelatin capsule).

Water-immiscible solvents may be mixed with surfactants and emulsifiers, in order to effect the spontaneous formation of small or microscopic vehicle droplets (e.g. microemulsions) when the water-immiscible solvent/emulsifier vehicle is mixed with water, as in the gastrointestinal tract Such mixtures include mixtures of triglycerides, or mono- and di-glycerides, with polysorbates, e.g. mixtures of Capmul®-MCM and polysorbate-80, or mixtures of Miglyol®-812 and polysorbate-80, in ratios of from 99/1 to 50/50, respectively. Further useful mixtures include mixtures of mono-, di-, and triglycerides with polysorbates, e.g. Capmul®-MCM/Miglyol®-812/polysorbate-80, in which Capmul®-MCM makes up 40-80% of the vehicle, with the remainder being any combination of Miglyol®-812 and polysorbate-80. Further useful mixtures include a vegetable oil and a surfactant, e.g. olive oil/polysorbate-80 in ratios of 99:1 to 50:50, or com oil/Labrafil®-M-2125-CS in ratios of 99:1 to 50:50. Polyethyteneglycols and other water-miscible sertraline solvents, e.g. glycerin, ethanol, propylene glycol, may be induded in amounts up to 30% of the vehicle, in order to optimize sertraline solubility in the vehicle, or to improve the viscosity of the vehicle to aid in capsule filling.

Solutions of sertraline in vehicles of the types described above are encapsulated in soft gelatin capsules, or are encapsulated in hard gelatin capsules. If encapsulated in hard gelatin capsules, it is preferred that the seam between the two capsule shell pieces be sealed, for example with a strip of gelatin, to prevent leakage. Encapsulation in soft-gelatin is well-known , and is described in "The Theory and Practice of Industrial Pharmacy", by L. Lachman, H. Lieberman, and J. Kanig, Lea and Febiger, publisher.

The pH-sensitive polymer may be any of those already disclosed, for example but not limited to cellulose acetate phthalate, polyvinylacetate phthalate, hydroxypropylmethylcellulose phthalate, copolymers of methacrylic acid and methylmethacrylate, and copolymers comprising acrylic acid and at least one acrylic acid ester.

Coating of sertraline-containing tablets, beads, capsules, and partides may be carried out using equipment known in the art. For example, sertraline-containing tablet cores and capsules may be coated with a pan-coater, such as a Hi-Coater (Freund Corp.), or an Accela-Cota (Manesty Corp., Liverpool). Sertraline-containing beads and particles are preferably coated using a fluidized bed coater, such as a Wurster coater, utilizing coating equipment available for example from the Glatt Corporation (Ramsey, NJ). Beads may also be coated using a rotary granulator, such as a CF-granulator available from Freund Corp.

Advantageously, because pH-triggered spatially-delayed devices possess a mechanism for sensing that the device has exited the stomach, interpatient variability in gastric emptying is not a significant issue.

### Spatially Delayed Enzyme-triggered Dosage Forms

In a further embodiment of a spatially-delayed sertraline dosage form, an "enzyme-triggered supported liquid membrane device" comprises sertraline formulated in a dosage form of the type described in lnternat Application PCT/US93/07463, published as WO 94/12159 on June 9, 1994, herein incorporated by reference. This embodiment generally has the form of an immediate-release tablet or multiparticulate (preferably a bead) containing sertraline plus carrier, a microporous hydrophobic membrane that at least partially, preferably entirely, surrounds the tablet or bead, and a hydrophobic liquid entrained within the pores of the membrane. Alternatively, the sertraline plus carrier may be incorporated into a capsule shell which comprises a microporous hydrophobic membrane with a hydrophobic liquid entrained within the pores of the capsule shell. The hydrophobic liquid is substantially impermeable to both the aqueous environment and the sertraline tablet or bead core formulation. The hydrophobic liquid is capable of change such that it becomes permeable to the aqueous environment or sertraline formulation. After ingestion of this embodiment by a mammal, including a human, sertraline release into the gastrointestinal system is delayed until the dosage form has exited the stomach and moved into the small intestine.

In a sertraline enzyme-triggered supported liquid membrane device, the entrained hydrophobic liquid is a liquid which undergoes change which is enzymatically catalyzed in the lumen of the small intestine, and not in the stomach, such that the pores become permeable to water and sertraline. The core can optionally contain an osmagent, swelling, or bursting material to help speed the release of sertraline once the dosage form has passed into the small intestine. Exemplary hydrophobic liquids are triglycerides, fatty anhydrides, fatty acid esters of cholesterol, hydrophobic amino add esters, and the like. Preferred triglycerides include triolein, tricaprylin, trilaurin, olive oil, palm oil, coconut oil, sesame seed oil, corn oil, peanut oil, soybean oil, and the like. Preferred fatty acid anhydrides include caprylic anhydride, lauric anhydride, myristic anhydride and the like. Mixtures of hydrophobic liquids may be used. Exemplary materials for the microporous hydrophobic support membrane include cellulose esters, polycarbonates, polyalkenes, polystyrenes, potyvinyl esters, polysiloxanes, polyacrylates, and polyethers. Preferably the hydrophobic microporous membrane with entrained hydrophobic liquid is impermeable to sertraline, until gastrointestinal enzymes have catalyzed a change in the hydrophobic oil, as described below.

In the environment of use, i.e., the small intestinal lumen, lipases and esterases degrade the aforementioned hydrophobic oils, releasing surfactant products in the pores of the microporous membrane of this embodiment, thus producing aqueous channels through which the sertraline in the device core may exit through the microporous hydrophobic support membrane. Release of the sertraline may occur by simple diffusion, osmotic pumping, osmotic bursting, or by bursting due to the presence of a swellable material, e.g., hydrogel, in the sertrafine-containing core of the device.

In a sertraline enzyme-triggered supported liquid membrane device as disclosed above, hydrophobic oils may be used which are substrates for small intestinal proteases such as carboxypeptidase and chymotrypsin. Exemplary oils are hydrophobic esters of amino acid derivatives.

In a further embodiment of a spatially-delayed sertraline dosage form, sertraline tablets, capsules, beads, or powders are coated with a coating which contains components which are enzymatically degraded by enzymes in the rumen of the small intestine, but not in the gastric lumen. The coating comprises waxes or triglycerides of natural or synthetic origin which are solid at body temperature. In preferred embodiments, 2-20% of a material which is liquid at body temperature, and which is degraded by small intestinal enzymes (e.g. trypsin, chymatrypsin, elastase, lipase), is included. Suitable enzymatically-labile liquids are those described above for "enzyme triggered suported liquid membrane devices". Preferred waxy coatings are applied at 3-20% of the weight of the uncoated sertraline tablet, capsule, bead, or powder.

### Temporally-delayed Dosage Forms

In a first embodiment of a temporally-delayed sertraline dosage form, a "bursting osmotic core device", sertraline is incorporated in an osmotic bursting device which comprises a tablet core or bead core comprising sertraline and one or more osmagents. Devices of this type have been generally disclosed in Baker, US 3,952,741, which is incorporated herein by reference. Examples of osmagents are sugars such as glucose, sucrose, mannitol, lactose, and the like; and salts such as sodium chloride, potassium chloride, sodium carbonate, and the like; water-soluble acids such as tartaric acid, fumaric acid, and the like. The sertraline-containing tablet core or bead core is coated with a polymer which forms a semipermeable membrane, that is, a membrane which is permeable to water but is impermeable to sertraline. Examples of polymers which provide a semipermeable membrane are cellulose acetate, cellulose acetate butyrate, and ethylcellulose, preferably cellulose acetate. A melt mixture of a polyethylene glycol, e.g., polyethylene glycol-6000, and a hydrogenated oil, e.g., hydrogenated castor oil, may be used as a coating, as described for isoniazid tablets by Yoshino (Capsugel Symposia Series; Current Status on Targeted Drug Delivery to the Gastrointestinal Tract; 1993; pp.185-190). Preferred semipermeable coating materials are cellulose esters and cellulose ethers, polyacrylic acid derivatives such as polyacrylates and polyacrylate esters, and polyvinyl alcohols and polyalkenes such as ethylene vinyl alcohol copolymer. Especially preferred semipermeable coating materials are cellulose acetate and cellulose acetate butyrate.

When a coated tablet or bead of a bursting osmotic core embodiment is placed in an aqueous environment of use, water passes through the semipermeable membrane into the core, dissolving a portion of the sertraline and osmagent, generating a colloidal osmotic pressure which results in bursting of the semipermeable membrane and release of sertraline into the aqueous environment. By choice of bead or tablet core size and geometry, identity and quantity of osmagent, and thickness of the semipermeable membrane, the time lag between placement of the dosage form into the aqueous environment of use and release of the enclosed sertraline may be tailored. It will be appreciated by those skilled in the art that increasing the surface-to-volume ratio of the dosage form, and increasing the osmotic activity of the osmagent serve to decrease the time lag, whereas increasing the thickness of the coating will increase the time lag. Preferred osmotic-bursting devices of this invention are those which exhibit substantially no release of sertraline (i.e. less than 10%) from the dosage form until the dosage form has exited the stomach, thus assuring that minimal sertraline is released in the stomach. A bursting osmotic core tablet or bead has a tablet or bead core which may contain from 15-80% sertraline, 5-60% osmagent, as described above, and 5-20% other pharmaceutical aids such as binders and lubricants. The semipermeable membrane coating on a tablet, preferably a cellulose acetate coating, is present at a weight corresponding to from 2% to 30%, preferably from 3% to 20%, of the weight of the tablet core. The semipermeable membrane coating on a bead, preferably a cellulose acetate coating, is present at a weight corresponding to from 2% to 80%, preferably from 3% to 40%, of the weight of the bead core.

A bursting osmotic core device possesses no mechanism for sensing that the device has exited the stomach and entered the small intestine. Thus devices of this type are temporally-delayed devices, that is, devices which release sertraline at a predetermined time after entering an aqueous environment, i.e., after being swallowed. In the fasted state, indigestible non-disintegrating solids, such as the "bursting osmotic core devices" of this invention, are emptied from the stomach during phase III of the Interdigestive Migrating Myoelectric Complex (IMMC), which occurs approximately every 2 hr in the human. Depending on the stage of the IMMC at the time of dosing in the fasted state, a bursting osmotic core device may exit the stomach almost immediately after dosing, or as long as 2 hr after dosing. In the fed state, indigestible non-disintegrating solids, which are <11 mm in diameter, will empty slowly from the stomach with the contents of the meal (Khosla and Davis, Int. J. Pharmaceut. 62 (1990) R9-R11). If the indigestible non-disintegrating solid is greater than 11 mm in diameter, i.e., about the size of a typical tablet, it will be retained in the stomach for the duration of the digestion of the meal, and will exit from the stomach during phase III of an IMMC, after the entire meal has been digested and has exited the stomach. A bursting osmotic core device which releases sertraline 10 minutes to 2 hour after ingestion decreases the sertraline Tₘₐₓ and also the incidence and severity of nausea, regurgitation, and diarrhea in a population of patients administered sertraline in such devices. A preferred bursting osmotic core device starts to release sertraline at 15 minutes to 1.5 hour after entering an aqueous environment, i.e., after ingestion, to more reliably assure that the device releases its sertraline distal to the stomach, when dosed in the fasted state.

In a further embodiment of a temporally-delayed sertraline dosage form, a "bursting coated swelling core", a sertraline-containing tablet or bead is prepared which also comprises 25-70% of a swellable material, such as a swellable colloid (e.g., gelatin), as described in Milosovich, US 3,247,066, incorporated herein by reference. Preferred swelling core materials are hydrogels, i.e., hydrophilic polymers which take up water and swell, such as polyethylene oxides, polyacrylic acid derivatives such as polymethyl methacrylate, polyacrylamides, polyvinyl alcohol, poly-N-vinyl-2-pyrrolidone, carboxymethylcellulose, starches, and the like. Preferred swelling hydrogels for this embodiment are polyethylene oxides, crosslinked polyaaylates, and carboxymethylcellulose. The colloid/hydrogel-containing sertraline-containing core tablet or bead is coated, at least in part, by a semipermeable membrane. Examples of polymers which provide a semipermeable membrane are cellulose acetate and cellulose acetate butyrate,and ethylcellulose. A melt mixture of a polyethylene glycol, e.g., polyethylene glycol-6000, and a hydrogenated oil, e.g., hydrogenated castor oil, may be used as a coating, as described for isoniazid tablets by Yoshino (Capsugel Symposia Series; Current Status on Targeted Drug Delivery to the Gastrointestinal Tract; 1993; pp. 185-190). Preferred semipermeable coating materials are cellulose esters and cellulose ethers, polyacrylic acid derivatives such as polyacrylates and polyacrylate esters, and polyvinyl alcohols and polyalkenes such as ethylene vinyl alcohol copolymer. Especially preferred semipermeable coating materials are cellulose acetate and cellulose acetate butyrate.

When a coated tablet or bead having a bursting coated swelling core is placed in an aqueous environment of use, water passes through the semipermeable membrane into the core, swelling the core and resulting in bursting of the semipermeable membrane and release of sertraline into the aqueous environment. By choice of bead or tablet core size and geometry, identity and quantity of swelling agent, and thickness of the semipermeable membrane, the time lag between placement of the dosage form into the aqueous environment of use and release of the enclosed sertraline may be chosen. Preferred bursting coated swelling core devices of this invention are those which exhibit substantially no release of sertraline from the dosage form until the dosage form has exited the stomach, thus assuring that minimal sertraline is released in the stomach.

A bursting coated swelling core tablet or bead has a tablet or bead core which may contain from 15-80% sertraline; 15-80% swelling material, e.g., hydrogel; 0-15% optional osmagent; and 5-20% other pharmaceutical aids such as binders and lubricants. The semipermeable membrane coating on a tablet, preferably a cellulose acetate coating, is present at a weight corresponding to from 2% to 30%, preferably from 3% to 20%, of the weight of the tablet core. The semipermeable membrane coating on a bead, preferably a cellulose acetate coating, is present at a weight corresponding to from 2% to 80%, preferably from 3% to 40%, of the weight of the bead core.

A bursting coated swelling core device possesses no mechanism for sensing that the device has exited the stomach and entered the small intestine. Thus devices of this type release their sertraline contents at a predetermined time after entering an aqueous environment, i.e., after being swallowed, as previously discussed for bursting osmotic core devices, and the same considerations and preferences apply to making bursting coated swelling core devices.

In a preferred embodiment of a temporally-delayed sertraline dosage form, immediate release sertraline tablets, beads, or particles are prepared to serve as cores which are coated with a water-soluble and/or water-disintegrable delay layer. Preferred delay coatings comprise hydroxypropylcellulose (HPC), hydroxypropylmethylcellulose (HPMC), polyethylene oxide, and polyvinyl pyrrolidone. For tablets this coating may be carried out in a tablet coating apparatus such as an HCT-30, HCT-60. or HCT-130 Coater (Freund Inc). The tablets are coated with an aqueous solution of HPMC or other appropriate polymer to a final coating weight of 5-50% of the final weight of the coated tablet. Heavier coating weights give longer delays before initiation of sertraline release into the use environment (the gastrointestinal lumen). The delay time may also be increased by incorporating small to moderate quantities of poorly water-soluble polymers (including but not limited to ethylcellulose (EC), cellulose acetate (CA), cellulose acetate butyrate) into the coating formulation. For example, the coating formulation may consist of 95:5 HPMC/EC to 50:50 HPMC/EC, or 95:5 HPMC/CA to 50:50 HPMC/CA. In the case of such mixed polymer coating systems, it may be necessary to adjust the solvent composition to dissolve the mixture of water-soluble and poorly water-soluble polymers. For example, mixtures of acetone and water, or ethanol and water, may be used as needed. Beads and particles may be similarly coated using a fluid bed coating apparatus, such as a GlattGPCG-5 coater. For beads, the coating comprises from 10% to 100% of the weight of the uncoated bead core. For sertraline particles, the coating comprises from 15% to 200% of the weight of the uncoated bead core.

In a further embodiment of a temporally-delayed sertraline dosage form, a solution or suspension of sertraline in a solvent is encapsulated in a soft or hard gelatin capsule which is then coated with a water-soluble and/or water-disintegratable polymer as described above for coating other types of cores. Useful and preferred solvents for sertraline include all those mentioned above for use with spatially dèlayed encapsulated solutions. The coating comprises polymers such as hydroxypropylcellulose, hydroxypropylmethylcellulose, polyethylene oxide, polyvinyl pyrrolidone, cellulose acetate, and ethylcellulose.

It will be appreciated by those skilled in the art that the various coated sertraline tablet, bead, and particle embodiments described above can be coated using standard coating equipment, such as pan coaters (e.g., Hi-Coater available from Freund Corp; Accela-Cota available from Manesty, Liverpool), fluidized bed coaters, e.g., Wurster coaters, (available from Glatt Corp, Ramsey, NJ and Aeromatic Corp., Columbia, MD), and rotary granulators, e.g., CF-Granulator (available from Freund Corp). Core tablets are made on standard tablet presses, such as a Kilian press. Sertraline containing beads and particles are made in fluidized bed granulators, rotary granulators, and extruder/spheronizers.

In preferred embodiments of this invention, sertraline dissolves quickly after a spatial delay or a temporal delay, in order to achieve as short a Tₘₐₓ as possible, for the purpose of hastening therapeutic efficacy. When formulating delayed release dosage forms of sertraline, it may be advantageous to employ a high solubility salt, a formulation which otherwise increases sertraline solubility, or a combination of both collectively referred to as a "high solubility form". Futhermore, inclusion of excipients which increase the sertraline dissolution rate is advantageous and prferred. Solubilizing agents and compositions are disclosed in co-pending provisional application [Pfizer Docket 9835JTJ], filed on even date herewith and herein incorporated by reference.

Whether due to the salt form employed or the particular excipients employed in the dosage form. the high solubility form should effect an aqueous sertraline solubility of at least 5 mg/ml. A high solubility salt or form is advantageous because (a) it dissolves more quickly than low solubility salts or forms (e.g., sertraline base, sertraline hydrochloride in the absence of solubilizing excipients), and (b) it provides a higher sertraline concentration in the gastrointestinal lumen, giving a higher sertraline concentration gradient across the intestinal wall, resulting in more rapid absorption of the sertraline dose.

Solubilization can be particularly important for sertraline dosage forms which are designed to minimize Tₘₐₓ, because some forms of sertraline, particularly high solubility salt forms, can form gels in many aqueous solutions, particularly those solutions which contain chloride ions, such as in the gastrointestinal tract.

Sertraline gels can be formed by simply introducing chloride ions into solutions of sertraline lactate or sertraline acetate. Similarily gels can be formed by introducing adds such as tartaric acid or combinations of acids and surfactants such as succinic acid and sodium lauryl sulfate to sertraline solutions. However, other adds and/or surfactant-like compounds can provide solubilizing effects, minimizing gel formation and providing a formulation basis for delivering sertraline into aqueous solutions containing chloride ions, such as intestinal fluids.

The gelling of sertraline in some forms is surprising, and the ability of certain additives to prevent this gelling is both surprising and unpredictable.

Thus, it is advantageous in delayed release formulations to utilize methods to solubilize sertraline quickly in the use environment. One method of providing more soluble sertraline is to make sertraline salts that have higher solubility, such as sertraline lactate, sertraline acetate, and sertraline aspartate. Preferred salts exhibit solubilities in water that are over 2 times greater than the sertraline HCI salt, which has a solubility of about 3 mgA/ml.

Another method of solubilizing sertraline is to use an agent, referred to herein as a "solubilizing agent", which actually functions to increase and preferably maintain the solubility of sertraline (or a salt thereof) in a use environment relative to the solubility of sertraline in the same use environment when the solubilizing agent is not present

Many solubilizing agents useful herein can be grouped into several broad categories:
1. Organic acids and organic add salts;
2. Partial Glycerides, i.e., less than fully esterified derivatives of glycerin, induding monoglycerides and diglycerides;
3. Glycerides;
4. Glyceride derivatives;
5. Polyethylene glycol esters;
6. Polypropylene glycol esters;
7. Polyhydric alcohol esters;
8. Polyoxyethylene ethers;
9. Sorbitan esters; and
10. Polyoxyethylene sorbitan esters.
11. Carbonate salts

The amount of solubilizing agent which should be employed depends on the particular solubilizing agent.

In the case of solubilizing agents which are organic acids the amount of solubilizer can be calculated as a ratio multiplied by the quantity of sertraline to be used, wherein the ratio is of organic acid solubility to solubility of sertraline salt: (organic acid or salt solubility/sertraline or sertraline salt solubility) x quantity of sertraline where the solubilities referred to are in mg/ml. The above expression is approximate, and some adjustment may be advantageous for optimization. Generally the above expression will give a quantity which is plus or minus 25% of the final value employed, although higher quantities of solubilizing agent can be incorporated without any particular additional advantage. In addition, organic acid salts can be added to modify the pH and/or solubility of the organic acid, effectively optimizing the solubilization effect of the agents.

For other types of solubilizing agents listed, typically the amount of solubilizing agent employed in the dosage form will be 1 to 150% by weight of the amount of sertraline employed therein, preferably 1 to 100%, more preferably 3 to 75%. Amounts of solubilizing agent higher than 150% may be employed, although it is believed that in most cases no particular advantage would be provided.

Examples of organic acids useful in the invention include malic, citric, erythorbic, adipic, glutamic, aspartic, maleic, aconitic, and ascorbic acid. Preferred acids are citric, erythorbic, ascorbic, glutamic, and aspartic. Salts of organic acids such as alkalkine earth metal (magnesium, calcium) salts and alkali metal (lithium, potassium, sodium) salts are also effective as well as mixtures of organic acids and their salts. Calcium salts such as calcium carbonate, calcium acetate, calcium ascorbate, calcium citrate, calcium gluconate monohydrate, calcium lactobionate, calcium gluceptate, calcium levulinate, calcium pantothenate, calcium proprionate, calcium phosphate dibasic, and calcium saccharate are preferred organic acid salts.

Examples of compounds within the other categories mentioned above are summarized in Table 1.

**Table 1**

| Solubilizing Agents | | |
|---|---|---|
| Class | Examples, Chemical Name | Examples, Trade Designation, (Vendor) |
| Partial Glycerides | Glyceryl Monocaprylate | Monocaprylin® (Sigma), Capmul® MCM(Abitec), Imwitor® 308 (Hüls) |
| | C8-C10 Partial Glycerides | Capmul® MCM (Abitec), Imwitor® 742 (Hüls), Imwitor® 988 (Hüls) |
| | Glyceryl Monooleate | Myverol® 18-99 (Eastman), Calgene® GMO (Calgene), Capmul® GMO(Abitec) |
| | Glyceryl Monolinoleate | Myverol® 18-92 (Eastman) |
| | Glyceryl Monostearate | Imwitor® 191 (Hüls) Calgene® GSO(Calgene) |
| | Glycery Monolaurate | Imwitor® 312 (Hüls) Calgene® GLO (Calgene) |
| | Glyceryl Dilaurate | Capmul® GDL (Abitec) |
| | | |
| Glycerides | Triacetin | Triacetin (Sigma) |
| | | |
| Glyceride Derivatives | PEG-Derivitized Glycerides | Cremophor® RH40, Cremophor® RH60 (BASF), Acconon CA5, CA-9, CA-15, W230, TGH (Abitec) |
| | Polyglycolized Glycerides | Gelucire® 44/14, 42/12, 50/13, 53/10, 35/1 48/09, 46107, 62/05, 50/02; Labrasol® (Gattefosse); Capmul® 3GO; 3GS, 6G2O, 6G2S, 10G4O, 10G 10O (Abitec) |
| | | |
| Polyethylene glycol Esters | PEG 200 Monolaurate, PEG 400 Monolaurate, PEG 600 Monolaurate | Calgene®20-L, Calgene® 40-L. Calgene® 60-L |
| | PEG 200 Monostearate, PEG 400 Monostearate, PEG 600 Monostearate | Calgene® 20-S, Calgene® 40-S, Calgene® 60-S |
| | PEG 200 Dilaurate, PEG 400 Dilaurate, PEG 600 Dilaurate | Calgene® 22-L, Calgene® 42-L, Calgene® 62-L |
| | | |
| Polypropylene Glycol Esters | Propylene Glycol Dicaprylate | Captex® 200 (Abitec) |
| | | |
| Polyhydric Alcohol Esters | Diethylene Glycol Monolaurate | Calgene® DGL |
| | Propylene Glycol Monolaurate | Calgene® PGML |
| | Ascorbyl Palmitate | Ascorbyl Palmitate (Sigma) |
| | | |
| Polyoxyethylene Ethers | PEG Lauryl Ether | Nonionic L-4 (Calgene) |
| | PEG Stearyl Ether | Nonionic S-20 (Calgene), Myrj 45, 52, 53, 59 (Sigma) |
| | | |
| Sorbitan Esters | Sorbitan Monolaurate | Catgene® SML, Span® 20 (Sigma) |
| | Sorbitan Monooleate | Calgene® SMO, Span® 80 (Sigma) |
| | | |
| Polyoxyethylene Sorbitan Esters | POE-20 Sorbitan Monolaurate | Calgene® PSML-20, Span® 20(Sigma), Tween® 20 (Sigma), Capmul® POE-L (Abitec) |
| | POE-20 Monooleate | Tween® 80, PSMO-20 |
| | | |
| Saccharide Esters | Sucrose Monolaurate | Ryoto LW-1540 (Chem Service) |
| | | |
| Phospholipids | Phosphatidyl choline | Lecithin (Sigma) |
| | Mixed phospholipids | Emphos D70-30C (Witco) |
| Block Copolymers | PEO-PPO Block Copolymers | Pluronic® F-68, F127, L-62 (BASF) |
| | | |
| Polyethylene Glycols | PEG 3350 | Various sources |

In addition other compounds useful as solubilizing agents in the invention are ethyl propionate, methyl paraben, propyl paraben, propyl gallate, niacinamide, ethyl vanillin, paraaminobenzoic acid, butylated hydroxyanisole, imidurea, and glycine. It is also noted that preferred compositions include mixtures of an organic acid with or without a corresponding organic acid salt, and one or more of the non-organic solubilizers listed above or in Table 1. It is also noted that it has generally been observed that in order to be most effective the solubilizer should have a solubility in the aqueous chloride-ion containing use environment of at least 1mg/ml, and preferably greater than 5mg/ml.

A preferred group of solubilizing agents, in addition to the preferred organic acids previously mentioned, includes those in Table 2.

**Table 2**

| Preferred Solubilizing Agents | | |
|---|---|---|
| Class | Examples, Chemical Name | Examples, Trade Names (source) |
| Partial Glycerides | Glyceryl monocaprylate | Monocaprylin (sigma), Capmul MCM(Abitec), Imwitor® 308 (Hüls) |
| | C8-C10 Partial Glycerides | Capmul® MCM (Abitec), Imwitor® 742 (Hüls), Imwitor® 988 (Hüls) |
| | Glyceryl Monostearate | Imwitor® 191 (Hüls) Calgene GSO(Calgene) |
| | Glyceryl Monolaurate | Imwitor® 312 (Hüls) Calgene GLO (Calgene) |
| | | |
| Glycerides | Triacetin | Triacetin (Sigma) |
| | | |
| Sorbitan Esters | Sorbitan Monolaurate | Calgene® SML, Span® 20 (Sigma) |
| | Sorbitan Monooleate | Calgene® SMO, Span® 80 (Sigma) |
| | | |
| Phospholipids | Phosphatidyl choline | Lecithin (Sigma) |
| | Mixed phospholipids | Emphos® D70-30C (Witco) |
| | | |
| Block Copolymers | PEO-PPO Block Copolymers | Pluronic® F-68, F127, L-62 (BASF) |
| | | |
| Polyethylene Glycols | PEG 3350 | Various sources |
| Note: Commercial vendors shown above are as follows: | | |
| Abitec Corp. Janesville, WI | | |
| BASF, Parsippany, NJ | | |
| Calgene Chemical Inc. Skokie, IL | | |
| Chem Service, Inc., West Chester, PA | | |
| Hüls America, Piscataway, NJ | | |
| Sigma, St. Louis, MO | | |
| Witco, Houston, TX | | |

Preferred combinations of solubilizing agents include (1) an organic acid plus a salt of the same or a different organic acid, (2) an organic acid plus a non-ionic solubilizing agent such as any of those listed in Table 1, and (3) an organic acid plus a salt of the same or a different organic acid plus a non-ionic solubilizing agent.

Particularly preferred individual solubilizing agents include aspartic acid, glycerol monocaprylate, calcium acetate, ascorbic acid, citric acid, glutamic acid, and calcium carbonate. Aspartic acid, glycerol monocaprylate, and calcium acetate are most preferred.

Also preferred are combinations of the preferred acids and preferred surfactant-like compounds. A screening test useful for testing candidate solubilizers for use together with low solubility sertraline salts, such as sertraline hydrochloride, is set forth in the examples.

Preferred embodiments of delayed release formulations comprise a bead core or tablet core containing sertraline hydrochloride and one or more solubilizing acids; preferable maleic, L-aspartic, tartaric, L-glutamic, malic, citric, erythorbic, and adipic acids; more preferably malic, citric, erythorbic, and adipic adds.

Preferred embodiments of delayed release formulations comprise a bead core or tablet core containing sertraline lactate or sertraline acetate or sertraline aspartate and an acid such as ascorbic, erythorbic, citric, glutamic, or aspartic acid. More preferred embodiments incorporate sertraline lactate or sertraline acetate.

Another preferred embodiment of delayed release formulations comprises a core containing sertraline lactate or sertraline acetate, an acid such as ascorbic, erythorbic, citric, glutamic, or aspartic acid and a surfactant-like material such as partial glycerides, glycerides, sorbitan esters, phospholipids, polyethylene oxide-polypropylene oxide block co-polymers, and polyethylene glycols.

Another preferred embodiment of delayed release formulations comprises a core containing sertraline-lactate or sertraline-acetate, and a surfactant-like material such as partial glycerides, glycerides, sorbitan esters, phospholipids, polyethylene oxide-polypropylene oxide block co-polymers, and polyethylene glycols.

These "high solubility" cores are further coated with a spatial delay coating or a temporal delay coating, as described herein.

Spatially delayed release embodiments of the invention are solid dosage forms or encapsulated solutions for oral administration comprising sertraline and a pharmaceutically acceptable carrier, which release not more than 10% of their incorporated sertraline into a mammal's stomach, and which release 70% or more of the remaining incorporated sertraline within 1.5 hour after entering said mammal's small intestine. Temporally delayed release embodiments of this invention are solid dosage forms or encapsulated solutions for oral administration comprising sertraline and a pharmaceutically acceptable carrier, which exhibit a delay in sertraline release after ingestion of from 10 minutes to 2 hours, preferably from 15 minutes to 1.5 hours. Following the delay period the dosage form releases at least 70% of the remaining contained sertraline sertraline in immediate-release fashion (i.e., within 1.5 hours).

The timing of release of sertraline in the stomach or small intestine may be tested utilizing a variety of approaches including, but not limited to, x-ray evaluation, nuclear magnetic resonance imaging, gamma scintigraphy, or direct sampling of the gastric and duodenal contents via intubation. These tests are certainly feasible, but can be somewhat difficult to carry out in humans.

A more convenient test for a spatially delayed release embodiment of the current invention is a modified version of a two part *in vitro* dissolution test which is described in the 1995 US. Pharmacopeia (USP 23), Section [724], Subsection "Delayed Release (Enteric-coated) Articles - General Drug Release Standard" which incorporates a 2 hr test of sertraline release in a simulated gastric fluid ("acid test"), followed by a test of drug release in a simulated intestinal fluid ("neutral test"). For tablets and capsules which do not contain multiparticulates, or do not disintegrate rapidly into multiparticulates, stirring is effected using paddles at 100 rpm. For multiparticulates, whether dosed in capsules, tablets, or unit dose packets, stirring is also effected using paddles at 100 rpm. If gelatin capsules are used, then 0.1 mg/mL of the enzyme trypsin must be added to the (neutral test, second stage) buffer. This two stage *in vitro* test can be modified so that it is useful in evaluating spatially delayed embodiments of this invention, as now described.

For pH-triggered spatially-delayed embodiments, the *in vitro* test is carried out as described in the USP "Enteric Test", with the requirements that dosage forms of this invention (a) release no more than 10% of the incorporated sertraline during the 2 hour "acid" portion of the test, and (b) release 70% or more of the remaining incorporated sertraline within 1.5 hour in the "neutral" portion of the test. The acid portion of the test is carried out in 750 ml 0.1N HCI, for 2 hour. After 2 hour, 250 ml 0.2M tribasic sodium phosphate, containing 10 gm polysorbate-80, is added to the acid medium (containing the dosage form), and the pH is adjusted to pH 6.8, using either 2M HCI or 2M NaOH, as appropriate. Thus the volume of solution in the neutral portion is about 1 liter. The solubility of sertraline is low in the second stage phosphate buffer (pH 6.8). Thus 1% polysorbate-80 is added to the neutral (pH 6.8) phosphate medium to increase the sertraline solubility to provide "sink conditions" for dissolution.

For enzyme-triggered spatially-delayed release embodiments described in this disclosure, the test is carried out as described above for pH-triggered dosage forms with modification to account for the fact that release of sertraline is triggered by the presence of an enzyme - pancreatic lipase, esterase, or protease - in the small intestine. Thus an enzyme usually at a concentration of 5 mg/mL, suitable for enzymatic degradation corresponding to or of the same type as that which triggers release in the human small intestine, is employed in the *in vitro* test. For *in vitro* evaluation of lipase-triggered delayed release dosage forms, a lipase such as 5 mg/ml of porcine pancreatic lipase (Sigma Chem., St. Louis, MO) is included in the phosphate buffer dissolution medium for the second stage of the dissolution test. For esterase- or protease-triggered delayed release systems, appropriate esterases or proteases (e.g. pancreatic esterase, trypsin, chymotrypsin, elastase) are included in the second stage of the *in vitro* test, e.g. at 5 mg/ml.

If the esterase, protease, or lipase is denatured by polysorbate-80, then the first hour of the "neutral" phase is carried out in the presence of enzyme and absence of polysorbate-80. After 1 hr in the "neutral" phase, 10g of polysorbate-80 is added.

For temporally-delayed embodiments *in vitro* dissolution is carried out at 37°C using a USP dissolution apparatus, with paddles stirring at 100 rpm. The dissolution test medium is 900 ml acetate buffer (0.13M acetic acid) with 0.075M sodium chloride using potassium hydroxide to adjust pH to 4.0. If gelatin capsules are used, then 0.1 mg/ml trypsin is included in the dissolution medium. A dosage form according to the invention releases substantially no sertraline (about 1% or less release) during the first 10 minutes of the test. The dosage form releases not more than 10% of the total sertraline contained therein during a second period of up to 2 hours in the acid test medium. At least 70 % of the remaining sertraline is then released over a third period lasting 1.5 hours.

Testing conditions are as otherwise specified in the USP.

In carrying out the described *in vitro* tests, sertraline can be quantitated using a high pressure liquid chromatography assay using a reverse phase C-18 column, with UV detection at 230 nm, or using some other suitable quantifiable sertraline analysis method.

Preferred delayed release sertraline dosage forms of this invention, upon oral dosing, result in a decrease in Tₘₐₓ of 0.5 hr or more, preferably of 1 hour or more, or a decrease in the incidence or severity of nausea, diarrhea, or regurgitation. To test whether a dosage form decreases Tₘₐₓ, a cross-over clinical study can be carried out in a population, usually 12 or more, of healthy fasted human volunteers. One half of the group receives the test sertraline dosage form and one half of the group receives an immediate release sertraline dosage form (e.g. Zoloft® tablets) at the same dose. Blood is collected at appropriate times before- and post-dose, and the blood sertraline concentration is determined by an appropriate assay, as described in the examples below. After a wash-out period of at least one week, each group receives the alternate dosage form, and blood sertraline concentrations are determined as before. Tₘₐₓ (immediate release dosage form) minus Tₘₐₓ (test dosage form) is determined for each subject. These differences are then averaged to give an average Tₘₐₓ difference. If this value is greater than 0.5 hr, then the dosage form is a dosage form of this invention. Analysis of sertraline in blood can be conducted by quantifying sertraline in blood plasma, as fully disclosed below in Example 1.

Decrease in side effects can be determined as follows. Two parallel groups of fasted healthy human subjects (at least 15 subjects per group) are dosed 200 mg sertraline. One group receives this dose in the test dosage form, and the other receives the dose in an immediate release dosage form (e.g. two 100 mg Zoloft® tablets). This dosing is carried out in a blinded fashion, that is, each subject also receives a placebo version of the other dosage form along with the sertraline-containing dosage form. The placebo dosage forms should not contain any excipients which are known to cause or ameliorate nausea, regurgitation, or diarrhea. Every hour for 12 hours post-dose, the subjects fill out a questionnaire which asks the subject to rate severity for nausea, regurgitation, and diarrhea over the previous hour. A visual-analogue scale is used with a range of 0-10, with 0 indicating no effect and 10 indicating the worst possible effect. For each treatment (e.g. test or immediate release dosage form), for each side effect (e.g. regurgitation), for each subject, all the scores are added to provide a cumulative score for that side effect in that subject on that treatment. For each treatment (e.g. test dosage form), for each side effect (e.g. regurgitation), the cumulative scores for the 15 (or more) subjects are added, and then divided by the number of subjects on that treatment to give a mean cumulative score (MCS).

If the MCS is higher for the immediate release dosage form treatment than for the test dosage form treatment, for any one of the side effects nausea, regurgitation, or diarrhea, then the test dosage form is a dosage form of this invention.

For clarification, the following information is provided:
1. Specification of a quantity in percent (%) means percent by weight based on total weight, unless otherwise indicated.
2. "Eudrag®" is the registered trademark of Röhm Pharma GmbH, Germany for a family of enteric polymeric methacrylates.
3. "Opad®" is the registered trademark of Colorcon Inc., West Point, PA for a family of plasticized cellulose ethers which include hydroxypropyl methylcellulose, hydroxypropyl cellulose and methylcellulose that are supplied as powders for reconstitution in water.
4. "Use environment" means the *in vivo* aqueous environment of the gastrointestinal tract or the test medium of an invitro test as described above used to quantify sertraline release from a dosage form.

### Example 1

This example demonstrates that the absorption of sertraline differs when sertraline is dosed directly to various portions of the gastrointestinal tract. In particular, this example demonstrates that delivery of sertraline directly to the duodenum (upper portion of the small intestine) results in more rapid achievement of peak sertraline plasma levels, compared with conventional oral delivery to the stomach. This indicates that oral sertraline dosage forms which delay release of sertraline until the dosage form has exited the stomach and entered the duodenum will support faster absorption of sertraline into the blood than will dosage forms which do not exhibit such a delay.

Two groups of 6 volunteers (Groups A and B) each were dosed with 200 mg sertraline or placebo by different four-way crossover regimens. Dosing was via (1) oral tablets, or (2) infusion of a solution through a nasoenteric tube into the stomach, duodenum, or ileocecal region of the small intestine, or (3) infusion into the transverse colon via anal intubation.

On four different occasions, Group A received (1) oral sertraline immediate release tablets plus placebo solution infused into the stomach, or (2) oral placebo tablets plus sertraline solution infused into the stomach, or (3) oral placebo tablets plus sertraline infused into the small intestine at the ileocecal junction, or (4) oral placebo tablets plus placebo solution infused into the small intestine at the ileocecal junction. On four different occasions, Group B received (1) oral sertraline immediate release tablets plus placebo solution infused into the duodenum, or (2) oral placebo tablets plus sertraline solution infused into the duodenum, or (3) oral placebo tablets plus sertraline infused into the transverse colon, or (4) oral placebo tablets plus placebo solution infused into the transverse colon.

The oral sertraline dose was administered as two 100 mg tablets. The infusions were administered as a 2 mg/ml solution at a rate of 20 ml/min for 5 min.

Blood samples were withdrawn prior to dosing, and at 0.5, 1, 1.5, 2, 4, 6, 8, 10, 12, 16, 24, 36, 48, 72, 96, 120, 144, 192, and 240 hr post-dosing. Plasma sertraline concentrations were determined by extraction of sertraline from basic human plasma into methyl t-butyl ether, followed by derivatization to form the trifluoroacetyl adduct. Analysis was carried out by capillary gas chromatography with electron capture detection. Total systemic exposure to sertraline was determined by measuring the area under the plasma sertraline concentration vs. time curve (AUC) for each subject in a given group, and then by calculating a mean AUC for the group. Cₘₐₓ is the maximum observed plasma sertraline concentration achieved in a subject Tₘₐₓ is the time at which Cₘₐₓ is achieved. Plasma pharmacokinetic data for this example are presented in Table I.

Table I presents the observed average Cₘₐₓ, Tₘₐₓ, and AUC for the various dosing regimens. Infusion into the stomach gave Cₘₐₓ, Tₘₐₓ, and AUC values which were similar to those observed after oral dosing of tablets (Group A). This indicates that the technique of infusion does not in itself cause any substantive change in the pharmacokinetics of sertraline. Infusion into the duodenum gave Cₘₐₓ and AUC values which were similar to those observed after oral dosing of tablets. However, infusion into the duodenum gave a Tₘₐₓ which was surprisingly shorter than that observed after oral dosing of tablets (3.7 hr vs. 6.7 hr) (Group B).

The observation that infusion of a sertraline solution into the stomach gave a longer Tₘₐₓ (7.0 hr) than infusion into the duodenum (3.7 hr) may indicate that release of sertraline solution from the stomach through the pylorus into the duodenum is inhibited relative to the release of water from the stomach, which generally occurs with a half-time of emptying of about 10 minutes. While not wishing to be bound by theory, an explanation for this unexpected observation is that sertraline inhibits its own gastric emptying. An alternative theory is that sertraline in solution, starting at the low pH of the stomach, precipitates (perhaps as the free base) when it moves into the duodenum, and slowly redissolves, resulting in slow overall absorption. Alternatively, as described above in this disclosure, sertraline may form a slowly dissolving gel in the high chloride environment of the stomach. When sertraline solution is available in the small intestine (duodenum), it can be rapidly absorbed into the bloodstream, however.

**Table 1-1**

| Pharmacokinetics of 200mg sertraline delivered to various portions of the gastrointestinal tract. | | | |
|---|---|---|---|
| **GROUP A** | | | |
| **Dosing Route** | **C**_{**MAX**} **(ng/ml)** | **T**_{**MAX**} **(hr)** | **AUC**_{**O-LAST**} **(ng)** · **hr/ml)** |
| Oral Tablet | 39.9 | 7.0 | 1174.5 |
| Stomach Infusion | 35.6 | 7.0 | 923.1 |
| Ileocecal Infusion | 27.3 | 5.0 | 727.1 |

| **GROUP B** | | | |
|---|---|---|---|
| **Dosing Route** | **C**_{**MAX**} **(ng/ml)** | **T**_{**MAX**} **(hr)** | **AUC**_{**O-LAST**}**(ng·hr/ml)** |
| Oral Tablet | 44.7 | 6.7 | 1153.4 |
| Duodenal Infusion | 48.8 | 3.7 | 1270.3 |
| Colonic Infusion | 10.9 | 4.4 | 179.4 |

### Example 2

This example demonstrates that certain sertraline side effects (e.g. nausea, regurgitation, and diarrhea) are partially or primarily mediated by direct contact of orally dosed sertraline with the upper gastrointestinal tract, rather than mediated by the presence of sertraline in the systemic circulation after absorption. Bypassing the stomach by dosing sertraline orally in a dosage form which exhibits delayed sertraline release can thus ameliorate the locally mediated side effects of sertraline.

In a subset of a larger double-blind, randomized, placebo-controlled parallel group study, healthy male human subjects were divided into two groups (Study 1). Group A received a single 200 mg sertraline dose as two 100 mg sertraline tablets (Zoloft commercial 100 mg tablets) ("bolus dosing" group). The tablets were administered with 50 ml water. Group B received two placebo tablets. All subjects were dosed after an overnight fast.

Blood samples were withdrawn prior to dosing, and at 0.5, 1, 2, 3, 4, 5, 6, 7, 8, 9, 10, 11, 12, 13, 14, 15, 16, 18, 20, 22, 24, 36, 48, 72, 96, 120, 144, 168, 192, and 240 hr post-dosing. Plasma sertraline concentrations were determined by extraction of sertraline from basic human plasma into methyl t-butyl ether, followed by derivatization to form the trifluoroacetyl adduct. Analysis was carried out by capillary gas chromatography with electron capture detection. Total systemic exposure to sertraline was determined by measuring the area under the plasma sertraline concentration vs. time curve (AUC) for each subject in a given group, and then by calculating a mean AUC for the group. Cₘₐₓ is the maximum observed plasma sertraline concentration achieved in a subject. Tₘₐₓ is the time at which Cₘₐₓ is achieved. After the 200 mg sertraline dose, average Cₘₐₓ was 74 ng/ml, average Tₘₐₓ was 6 hr, and average AUC was 1646 ng-hr/ml (averaged for 15 subjects).

A similar second study was carried out (Study II). After the 200 mg sertraline dose, average Cₘₐₓ was 75 ng/ml, average Tₘₐₓ was 5.4 hr, and average AUC was 1744 ng-hr/ml (averaged for 11 subjects). Four subjects in the 200 mg dose group had emesis at 2.6, 2.8, 2.8, and 3.8 hr. The data from these four subjects were not included in the pharmacokinetic averages.

Prior to dosing and each blood sampling time, each subject filled out a questionnaire, which consisted of a series of "Visual Analogue Scales" in which the subject was required to rate, on a scale of 0-10, the severity of certain potential side effects. The subjects were instructed that "0" indicated an absent effect and "10" indicated the worst possible effect The subjects were instructed to interpolate between 0 and 10 for moderate side effects.

A total of 30 subjects completed Study I: 15 each in Groups A and B. For each side effect evaluated at 30 time points, a total of 900 individual visual-analogue-scale evaluations were obtained. A total of 29 subjects completed Study II: 14 in Group A and 15 in Group B. For each side effect evaluated at 30 time points, a total of 870 individual visual-analogue-scale evaluations were obtained.

Figure 1 presents the relationship between plasma sertraline concentration and average self-reported visual analogue score for nausea in Study I. This plot, known as a pharmacokinetic-pharmacodynamic relationship plot ("PK/PD Plot"), was obtained as follows. For the 15 subjects in Group A, plasma sertraline concentration was averaged at each blood collection timepoint, to give an average sertraline concentration for Group A at each time point. Likewise, for the 15 subjects in Group A, the visual analogue score for nausea was averaged at each time point The average nausea scores at each time point (y-axis) were plotted vs.sertraline plasma levels at the corresponding time point (x-axis). The arrow on the plot demonstrates the progression of the PK/PD relationship as time progressed. The PK/PD plot of Figure 1 exhibits "clockwise hysteresis" for the 200 mg bolus dose. Thus as time progressed, the nausea score and the plasma sertraline concentration both increased until the nausea score reached a maximum value, at a plasma sertraline concentration which was below the maximum plasma sertraline concentration Cₘₐₓ. As Cₘₐₓ was reached (at about70 ng/ml), the nausea score fell to a lower value. As the subsequent plasma sertraline concentrations fell, the nausea score assumed values which were lower than the scores observed for the same plasma sertraline concentrations at earlier timepoints. This "clockwise hysteresis" (or "proteresis") is consistent with the interpretation that sertraline-induced nausea is significantly mediated by direct contact of sertraline with the GI tract, and is not entirely mediated by the presence of sertraline in the systemic blood, since the average nausea score is not monotonically related to plasma sertraline concentration. At early time points after dosing (0-3 hr), orally dosed sertraline is primarily in contact with the stomach, and may inhibit its own emptying into the duodenum (described in Example I). Since nausea is not directly monotonically related to plasma sertraline concentration, and is apparently primarily mediated locally by contact with the gastrointestinal tract, releasing sertraline lower in the gastrointestinal tract, e.g. the duodenum or jejunum, will result in faster absorption and decreased contact time with the upper gastrointestinal tract, and thus less nausea.

In Study 1, diarrhea was also shown to exhibit clockwise hysteresis in its side effect score vs. plasma sertraline concentration curve. The maximum diarrhea score was reached at 3-hr post-dose, long before the observed average plasma Tₘₐₓ of 6 hr in these subjects. Thus delaying the release of orally dosed sertraline until the stomach is passed may result in less diarrhea.

As described above, in Study 2, four subjects exhibited regurgitation. Individual PK/PD plots for these subjects, for the side effect regurgitation, exhibited clockwise hysteresis. Thus delaying the release of orally dosed sertraline until the stomach is passed can result in less regurgitation.

### Example 3

This example illustrates a process for making a delayed release sertraline tablet The processing comprised (1) wet granulating sertraline with hydroxypropyl cellulose; (2) drying, milling and blending the granulation; (3) blending all the remaining components with the granulation except for magnesium stearate; (4) adding and blending magnesium stearate; (5) compressing the final blend into tablets; and (6) applying a pH-sensitive, delayed release coating onto the tablets. This example further illustrates the in vitro release profile of sertraline from enteric tablets using the in vitro test described in the specifications.

In a batch size of 4kg, sertraline was blended with hydroxypropyl cellulose (Klucel EF™, Aqualon) for 5 minutes in a suitable mixer. After the initial mixing time, water was added to the blend as a granulating agent while mixing was continued until the desired endpoint was reached. Next, the wet granulation was spread onto polyethylene-lined trays and dried in a 50°C oven until the percent moisture loss on drying, LOD, is less than 0.5%. The granulation was subsequently milled (Fitzpatrick JT Mill) and blended in a stainless steel twin-shelled blender for 10 minutes. Next, the remaining components except magnesium stearate were added to the blender and blended for 30 minutes. Then, magnesium stearate was added to the mixture and blended for 5 minutes. Using a Manesty Beta-Press (Manesty Machines, Liverpool, England), the final blend was compressed into 600mg tablets using 7/16 inch standard round concave tablet tooling punches. The composition of the uncoated tablets is listed in Table 3-1.

**Table 3-1.**

| Delayed Release Tablet Core Composition Manufactured on the Beta-Press with Dosage Strength of 200mgA/tablet. | | |
|---|---|---|
| COMPONENT | GRAMS/BATCH | %/TABLET |
| Example Number | | 12A |
| Sertraline Hydrochloride | 1492.1 | 37.3 |
| Hydroxypropyl Cellulose (a) | 120.0 | 3.0 |
| Calcium Phosphate (b) | 640.0 | 16.0 |
| Microcrystalline Cellulose (c) | 1197.9 | 30.0 |
| Sodium Starch Glycolate (d) | 500.0 | 12.5 |
| Magnesium Stearate | 50.0 | 1.2 |
| TOTAL: | 4000 grams | 600 mg |

| | | |
|---|---|---|
| (a) Hydroxypropyl Cellulose is Klucel® EF™, Aqualon | | |
| (b) Calcium Phosphate means dibasic calcium phosphate dihydrate, Emcompress®, Edward Mendell Co. Inc. | | |
| (c) Microcrystalline Cellulose is Avicel®PH101, FMC Corporation | | |
| (d) Sodium Starch Glycolate is Explotab®, Edward Mendell Co. Inc. | | |

Next, the sertraline core tablets were spray coated with a pH-sensitive, delayed release coating in a coating pan (Freund Model HCT-30, Vector Corporation, Marion, IA) until the desired end point (coating weight %) was achieved. The delayed release coating was applied from a suspension containing 16.0% methacrylic acid copolymers (Eudragit® L 30 D-55, Rohm Pharma), 4.0% talc as detackifying agent, 1.6% triethyl citrate as plasticizer and 78.4% water. A 6% and a 10% coating was applied to the core tablets (Table 3-2).

**Table 3-2.**

| Composition of Delayed Release Sertraline Tablets Containing the Core Formulation from Coated with a pH-Sensitive Coating to 6 and 10%. | | |
|---|---|---|
| COMPONENT | mg Solid Per Tablet | mg Solid Per Tablet |
| Coating Level (wt %) | 6% | 10% |
| CORE TABLET | | |
| Sertraline Hydrochloride | 223.8 | 223.8 |
| Hydroxylpropyl Cellulose (a) | 18.0 | 18.0 |
| Calcium Phosphate (b) | 96.0 | 96.0 |
| Microcrystalline Cellulose (c) | 180.0 | 180.0 |
| Sodium Starch Glycolate (d) | 75.0 | 75.0 |
| Magnesium Stearate | 7.2 | 7.2 |
| Total Core Weight | 600 mg | 600 mg |

| COATING | 6% | 10% |
|---|---|---|
| Eudragit L30 D-55(a) | 26.7 | 44.5 |
| Talc | 6.7 | 11.1 |
| Triethyl Citrate | 2.6 | 4.4 |
| Total Coating Weight | 36 mg | 60 mg |

| | | |
|---|---|---|
| (a) Eudragit® L30D-55 is composed of 30% aqueous dispersion | | |

Delayed release tablets from Example 3A containing a 10% pH-sensitive coating, as shown in Table 3-3, were tested using the in vitro delayed release dissolution test procedure with quantification by reverse-phase high performance liquid chromatography (HPLC) analysis for sertraline to determine sertraline released as a percentage of the total dose, as described below.

Delayed release dosage forms of sertraline were tested in a standard USP rotating paddle apparatus as disclosed in United States Pharmacopeia XXIII (USP) Dissolution Test Chapter 711, Apparatus 2. The delayed release dissolution test procedure involved paddle rotation of 100rpm and the dissolution was conducted in two stages, all media maintained at 37°C with covered vessels to prevent evaporation. The first stage, the acid phase, was implemented by placing the dosage form into 750 mL of 0.1 N HCl for two hours, at which time an aliquot (typically 2 or 10mL) from the test medium was withdrawn and analyzed for sertraline by the HPLC assay described below. The second stage, the buffered phase with solubilizer, was achieved by adding 250 mL of 0.2M tribasic sodium phosphate containing an additional 10 grams of polysorbate 80 to the acid phase and adjusting the pH to 6.8 using either 2M hydrochloric acid or 2M sodium hydroxide. Thus, converting the acid phase from the first stage to a buffer having a pH of 6.8 and 1% solubilizer in the second stage. At intervals after addition of the phosphate buffer, filtered aliquots (typically 2 or 10mL) of test medium were withdrawn and analyzed for sertraline by HPLC as described below.

Sertraline quantification was conducted by reverse-phase high performance liquid chromatography as follows. A fixed volume of 20 µL was injected onto the analytical column (150 mm length x 3.9 mm diameter Nova-Pac C-18 column). The isocratic mobile phase consisted of an aqueous acetate buffer, methanol and acetonitrile in volume percentages of 40/15/45. The aqueous acetate buffer was prepared by the following: (1) 2.86 mL of glacial acetic acid was added to a 1000 mL Erlenmeyer flask with a magnetic stir bar in an ice bath; (2) while stirring, 3.48 mL of triethylamine was added to the flask; and (3) the flask was filled to volume and mixed well. To the aqueous acetate buffer (40 %) was added HPLC-grade methanol (15 % v/v) and HPLC-grade acetonitrile (45 % v/v). After mixing well, the mobile phase was vacuum filtered and degassed using a 0.45µm PTFE filter (Lid-X 305 disposable solid liquid separators). The mobile phase flow rate was 1.8 mL/min with sertraline UV detection at 254nm.

The results are presented in Table 3-3 for the tablet coated with 10% (data represent the average of three separate tests at 200mgA/unit, n=3). This example satisfies the dissolution criteria and is a delayed release embodiment of this invention.

**Table 3-3.**

| In Vitro Sertraline Delayed Release from Enteric Coated Tablets into 750mL 0.1 N HCI for 2 hours and then 1000mL Enteric Buffer Media with 1% Tween-80, pH 6.8, at 37°C in USP Apparatus #2 with Paddle Speed Setting of 100rpm (n=3 tablets). | | | | |
|---|---|---|---|---|
| Example | Enteric Coating (%) | Acid Phase Q₂ (%) | Buffered Phase Q_{3.5} (%) | → Q₆ (%) |
| 3 | 10 | 8.1 | 93.3 | 97.1 |
| Q2 is % released at 2hr; Q3.5 is % released at 3.5hr. | | | | |

### Example 4

This example illustrates a process for making multiparticulates for use in making delayed-release dosage forms designed to release sertraline predominantly below the stomach. The process comprises (1) preparing uncoated sertraline multiparticulate cores; (2) applying a pH-sensitive, delayed release coating.

Sertraline-containing multiparticulate cores are prepared by blending sertraline compound with microcrystalline cellulose (Avicel® PH101, FMC Corp., Philadelphia, PA) in relative amounts of 85:15 (w/w), wet massing the blend in a Hobart mixer with water equivalent to approximately 27 % of the weight of the blend, extruding the wet mass through a perforated plate (Luwa EXKS-1 extruder, Fuji Paudal Co., Osaka Japan), spheronizing the extrudate (Luwa QJ-230 marumenzer, Fuji Paudal Co.) and drying the final cores which are about 1 mm diameter.

Then a Wurster bottom spray fluid bed processor (Glatt GPCG-1) is used to apply a delayed release coating. Typical delayed release coating levels are 5 % to 50 % in order to be sure that the delayed release dissolution criterion are met. The delayed-release coating is a suspension containing 12.3 % methacrylic add copolymers (Eudragit® L 30 D-55), 6.2 % talc, 1.5 % triethyl citrate and 80 % water.

Because the delayed release coating is soluble in environments where the pH is greater than 5.5, the multiparticulates thus prepared release sertraline from the coated particle cores below the stomach where the pH is greater than 5.5.

### Example 5

This example illustrates a process for making multiparticulates for use in making delayed-release dosage forms designed to release sertraline predominantly below the stomach. The process comprises (1) preparing uncoated sertraline multiparticulate cores; (2) applying a protective coat over the core particles; and (3) applying a second, pH-sensitive, delayed release coating over the first coat.

Multiparticulate cores containing drug are prepared using a fluid bed processor with rotor insert (Model GPCG-1). The rotor bowl is initially charged with 400 g of sertraline drug and a binder solution containing 5 % poly(ethyl acrylate, methyl acrylate)(Eudragit® NE-30-D), 5 % plasticized hydroxypropyl methylcellulose (Opadry®) and 90 % water is sprayed into the rotating bed until an average core granule size of about 250 µm is achieved.

Onto the uncoated core particles in the same fluid bed processor with rotor insert, a binder solution containing 5 % plasticized hydroxypropyl methylcellulose (Opadry®) solution is sprayed until a coating of 10 % is applied. This intermediate coating enhances the adhesion to the core particles of the final delayed release coating.

A delayed release coating (typically 5 % to 50 % is required to meet the delayed release criterion) is applied using the same fluid bed processor as above. The delayed-release coating is a suspension containing 12.3 % methacrylic acid copolymers (Eudragit® L 30 D-55), 6.2 % talc, 1.5 % triethyl citrate and 80 % water. The final product is a delayed-release multiparticulate with particles having an average size of about 300 µm.

### Example 6

This example illustrates the preparation of a pH-triggered spatially delayed sertraline coated tablet with a Cellulose Acetate Phthalate Coat.

Sertraline tablet cores are manufactured according to the formula described in Table 3-1 of Example 3, using the procedure described in Example 3. Tablet cores are then spray-coated with an acetone solution of cellulose acetate phthalate (CAP) in a HCT-60 Hi-Coater® spray-coating apparatus (Freund Ind. Corp., Tokyo). The CAP is plasticized with 25% (by weight) diethylphthalate (DEP). Sufficient CAP is sprayed onto the tablets to result in a final coating polymer weight, after drying, of 5-50 wt%, relative to the weight of the uncoated tablet bed.

### Example 7

This example illustrate the preparation of a pH-triggered spatially delayed CAP-coated sertraline tablet with a barrier coat.

Sertraline tablet cores are manufactured according to the formula described in Table 3-1 of Example 3, using the procedure described in Example 3. Tablets are spray coated with a solution of hydroxypropylmethylcellulose (HPMC; Colorcon, Inc.) in water, using a HCT-60 Hi-Coater®. In this manner, tablets are coated with a 5 wt% barrier coat of HPMC, relative to the initial tablet weight. Tablets are then further spray-coated with cellulose acetate phthalate (CAP) and DEP plasticizer (as described in Example 7), in the HCT-60 Hi-Coater. Sufficient CAP is sprayed onto the tablets to result in a final coating polymer weight, after drying , of 5-50 wt%, relative to the weight of the uncoated tablet. The HPMC coat serves as a barrier between sertraline and the pH-sensitive CAP coat. This barrier coat prevents premature dissolution (or weakening) of the CAP coat, e.g., in the low pH environment of the stomach, potentially caused by a locally higher pH in the tablet interior due to the presence of sertraline.

### Example 8

This example illustrates the preparation of a pH-triggered spatially-delayed acrylic resin-coated sertraline tablet with a barrier coat

Sertraline tablet cores are manufactured according to the formula described in Table 3-1 of Example 3, using the procedure described in Example 3. Tablets are spray coated with a solution of hydroxypropylmethylcellulose (HPMC) (Colorcon, Inc.) in water, using a HCT-60 Hi-Coater. In this manner, tablets are coated with a 5 wt% barrier coat of HPMC, relative to the initial tablet weight. A coating formulation is prepared according to the formulation in Table 3-3.

The coating solution is sprayed onto HPMC-coated sertraline tablet cores using a Freund HCT-30 Hi-Coater.

Thè total acrylic resin polymer weight applied is 5-50% of the weight of the uncoated tablet bed. The HPMC undercoat serves as a barrier between sertraline and the pH-sensitive acrylic resin coat. This barrier coat prevents premature dissolution (or weakening) of the acrylic resin coat, e.g., in the low pH environment of the stomach, potentially caused by a locally higher pH in the tablet interior due to the presence of sertraline.

### Example 9

This example illustrates preparation of a temporally-delayed (water-activated) sertraline tablet dosage form.

Sertraline tablet cores are manufactured according to the formula described in Table 3-1 of Example 3, using the procedure described in Example 3. Tablets are then coated with a water-soluble and/or water-disintegrable delay layer, in a tablet coating apparatus such as an HCT-30, HCT-60, or HCT-130 Coater (Freund Inc). The tablets are coated with an aqueous solution of HPMC to a final coating weight of 5-50% of the final weight of the coated tablet Heavier coating weights give longer delays before initiation of sertraline release into the use environment (the gastrointestinal lumen). The delay time may also be increased by incorporating small to moderate quantities of poorly water-soluble polymers (including but not limited to ethylcellulose (EC), cellulose acetate (CA), cellulose acetate butyrate) into the coating formulation. For example, the coating formulation may consist of 95:5 HPMC/EC to 50:50 HPMC/EC, or 95:5 HPMC/CA to 50:50 HPMC/CA. In the case of such mixed polymer coating systems, it may be necessary to adjust the solvent composition to dissolve the mixture of water-soluble and poorly water-soluble polymers. For example, mixtures of acetone and water, or ethanol and water, may be used as needed. In the environment of use, the dosage forms of this example exhibit a delay in sertraline release, during which time the coating polymer dissolves from the sertraline tablet core surface. After the delay, the sertraline core tablet releases at least 70% of its remaining incorporated sertraline in 1.5 hours.

### Example 10

This example illustrates that organic acids have the ability to raise the solubility of the hydrochloride salt of sertraline. The acids were screened by dissolving the candidate add in water and then stirring excess sertraline hydrochloride in the acid solution for at least 8 hours. The concentration of sertraline in the supematant was then measured by HPLC analysis. The results of this test are listed in Table 10-1, below. Most of the acids listed in the table successfully raised the solubility of sertraline hydrochloride (normal solubility 2.5 mg/ml).

**Table 10-1**

| Excipient | Approximate Excipient Concentration (mg/ml) | Sertraline Solubility (mg/ml) |
|---|---|---|
| D,L-malic acid | 900 | 21 |
| Citric acid | 600 | 20 |
| Erythorbic acid | 400 | 19 |
| Adipic acid | 14 | 12 |
| Maleic acid | 700 | 6.4 |
| L-aspartic acid | 10 | 5.5 |
| Tartaric acid | 1400 | 5.5 |
| L-glutamic add | 12 | 5.4 |
| Fumaric acid | 11 | 3.1 |
| Tannic acid | 2000 | 2.8 |
| D,L-tyrosine | 600 | 2.2 |

Preferred acids, based on this screening test, are malic, citric, erythorbic, and adipic acids. Maleic, L-aspartic, tartaric, and L-glutamic acids also significantly improved sertraline hydrochloride solubility. Delayed-release dosage forms with such adds in the core will perform better than those without such adds.

### Example 11

This example illustrates that organic acids have the ability to raise the solubility of the acetate salt of Sertraline by a method similar to that used for the hydrochloride salt described in Example 10. The excipient, excipient concentration, and sertraline solubility are listed in Table 11-1 below. Based on these results, preferred adds to include in a dosage form where increased sertraline acetate solubility is desired are ascorbic, erythorbic, citric, lactic, aspartic, glutamic, and aconitic acids.

**Table 11-1**

| Excipient | Excipient Concentration (mg/ml) | Sertraline Solubility (mg/ml) |
|---|---|---|
| Ascorbic acid | 400 | >425 |
| Erythorbic acid | 400 | >330 |
| Citric acid | 600 | 146 |
| Lactic acid | 213 | >294 |
| Aspartic acid | 7 | 110 |
| Glutamic acid | 12 | 108 |
| Aconitic acid | 500 | >92 |
| Itaconic acid | 150 | 72 |
| Succinic acid | 77 | 28 |
| None | - | 64 |

### Example 12

This example illustrates that organic acids and three calcium salts have the ability to raise the aqueous solubility of the lactate salt of sertraline using a method similar to that used for the hydrochloride salt described in Example 10. The excipient, the excipient concentration in the aqueous test solution, and the sertraline lactate solubility in the test solution are listed in Table 12-1 below. Solubility of sertraline lactate in water is approximately 125 mg/ml. The data below show that eight organic acid solutions had sertraline lactate solubilities of about the same or higher than 125 mg/ml; adipic, erythorbic, itaconic, citric, aspartic, glutamic, histidine, and ascorbic. Also, a solution of a mixture of two of these adds also had high solubility; ascorbic and aspartic. Sertraline lactate solubility was also high in calcium salt solutions, either alone (calcium citrate) or mixed with ascorbic acid.

**Table 12-1**

| Excipient | Excipient Concentration (mg/ml) | Sertraline Lactate Solubility (mg/ml) |
|---|---|---|
| Adipic acid | 14 | 360 |
| Erythorbic acid | 400 | >217 |
| Itaconic acid | 150 | >202 |
| Citric acid | 600 | 162 |
| Aspartic acid | 7 | >155 |
| Glutamic acid | 12 | >125 |
| Histidine | 42 | >116 |
| Ascorbic/Aspartic | 400/7 | 116 |
| Ascorbic | 400 | 102 |
| Glycine | 250 | 66 |
| Aconitic acid | 200 | <59 |
| Tartaric add | 1400 | 12 |
| Fumaric acid | 11 | <9 |
| Sorbic acid | 3 | <9 |
| Calcium lactate/ Ascorbic acid | 50/400 | 160 |
| Calcium citrate | 10 | 165 |
| Calcium carbonate/ Ascorbic acid | 50/400 | 176 |
| None | - | 125 |

### Example 13

The lower solubility of the sertraline chloride salt and of all sertraline lactate and sertraline acetate salts in the presence of high chloride concentrations suggest that core formulations are preferred for which sertraline stays in solution that is, it does not precipitate or form a gel-like material when chloride is present in the use environment. Certain organic acids and salts were found to inhibit precipitation or gelation of sertaline when chloride is present via the following screening test. Sertraline lactate was dissolved in water either alone (as a control) or with a candidate excipient. Sodium chloride was then added (as a concentrated solution) and the result observed. An excipient was considered beneficial if the solution remained clear and fluid. The more chloride that could be added to an excipient solution with the solution remaining dear, the more beneficial was the excipient. Table 13-1 below shows the results of this screening test, indicating that all the excipients tested increased sertraline concentration in the chloride solutions.

**Table 13-1**

| Excipient | Excipient Concentration (mg/ml) | Concentration NaCI (mM) | Final Sertraline Concentration (mg/ml) | Observation After NaCI Addition |
|---|---|---|---|---|
| None | - | 38 | 22 | gel/precipitate |
| Ascorbic/ Aspartic acids | 400/7 | 152 | 162 | solution |
| Aspartic acid | 7 7 | 114 152 | 162 100 | solution gel |
| Ascorbic acid | 400 | 100 | 102 | predpitate |
| Ascorbic acid/ calcium lactate | 400/50 | 150 | 165 | solution |
| Ascorbic acid/ calcium carbonate | 400/50 | 150 | 170 | slightly turbid |
| Citric acid/ calcium lactate | 600/50 | 150 | 162 | solution |
| Histidine | 42 | 150 | 110 | slight precipitate |

### Example 14

Organic compounds (solubilizers) were screened for their ability to enhance the solubility of sertraline salts in aqueous solutions with or without the presence of chloride. Excess sertraline lactate was added to an aqueous solution of the candidate solubilizer and, in most cases an organic acid. The organic acids were saturated in these solutions also, and the additional solubilizing agents were at the concentrations shown in Table 14-1. The equilibrium sertraline solubility was measured. Then, sodium chloride was added to the saturated solution and the final sertraline concentration was measured. The results of these screening tests are summarized in Table 14-1.

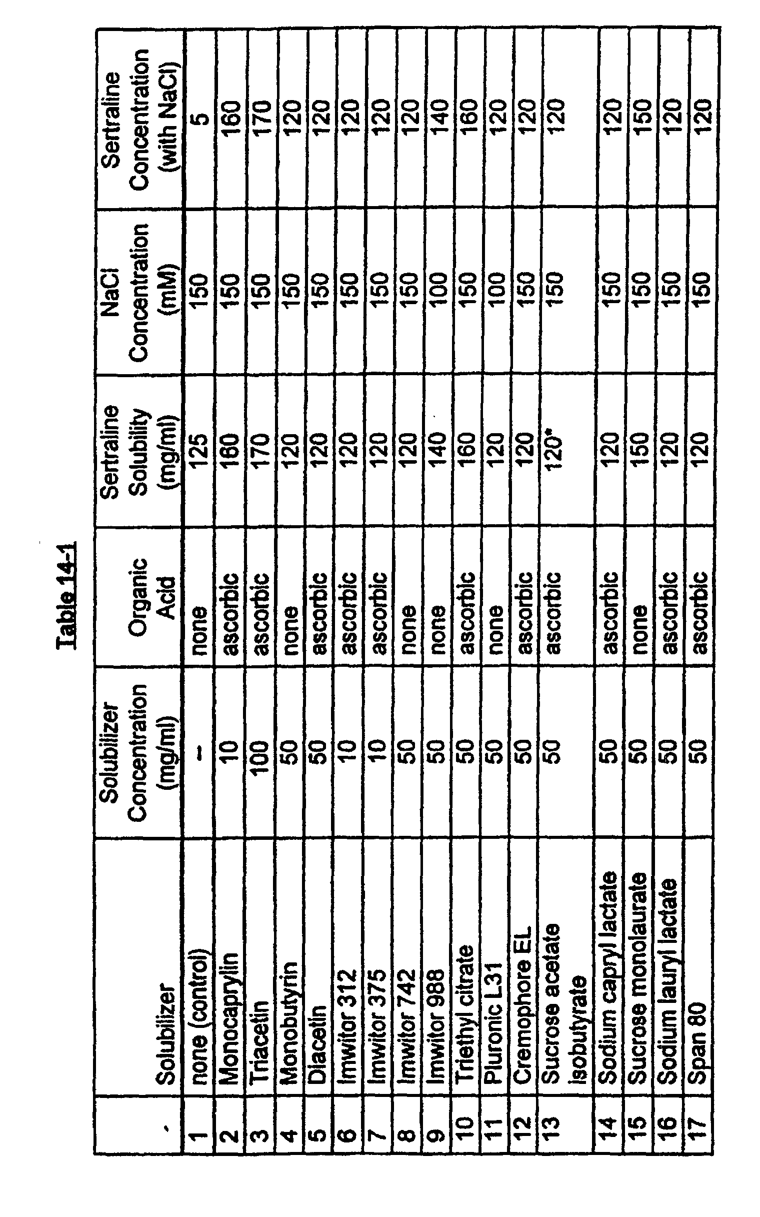

### Example 15

This example illustrates that solubilizers for sertraline also can increase the rate of dissolution of sertraline. The effect of a candidate excipient on sertraline dissolution rate was determined by adding solid drug, the candidate solubilizing excipient, and, in some cases, other excipients such as an organic acid and an osmagent (such as a sugar) to a 1.8 ml centrifuge tube. The sample tubes were spun at 14K G for 5 minutes in a microcentrifuge to pack the powder. 150 µl gastric buffer was added to the packed powder and the samples were gently agitated, then spun at 14K G in a microcentrifuge for 2 minutes. The samples were then removed from the micxocentrifuge and allowed to stand undisturbed until the solution was removed. The solution was removed from the samples after a total of 10 minutes after gastric buffer was added to the powder pack, and analyzed by HPLC to determine the sertraline concentration.

The dissolution rate (mg sertraline/ml-min) was calculated from the measured concentration of dissolved sertraline in the supernatant as a function of time over the first 10 minutes of dissolution. These dissolution rates and the excipient mixtures for which they were measured are summarized in Table 15-1 below. As shown, several excipient mixtures containing solubilizers significantly (about 3X or greater) increased the dissolution rate of sertraline, compared with sertraline alone and compared with sertraline and ascorbic acid.

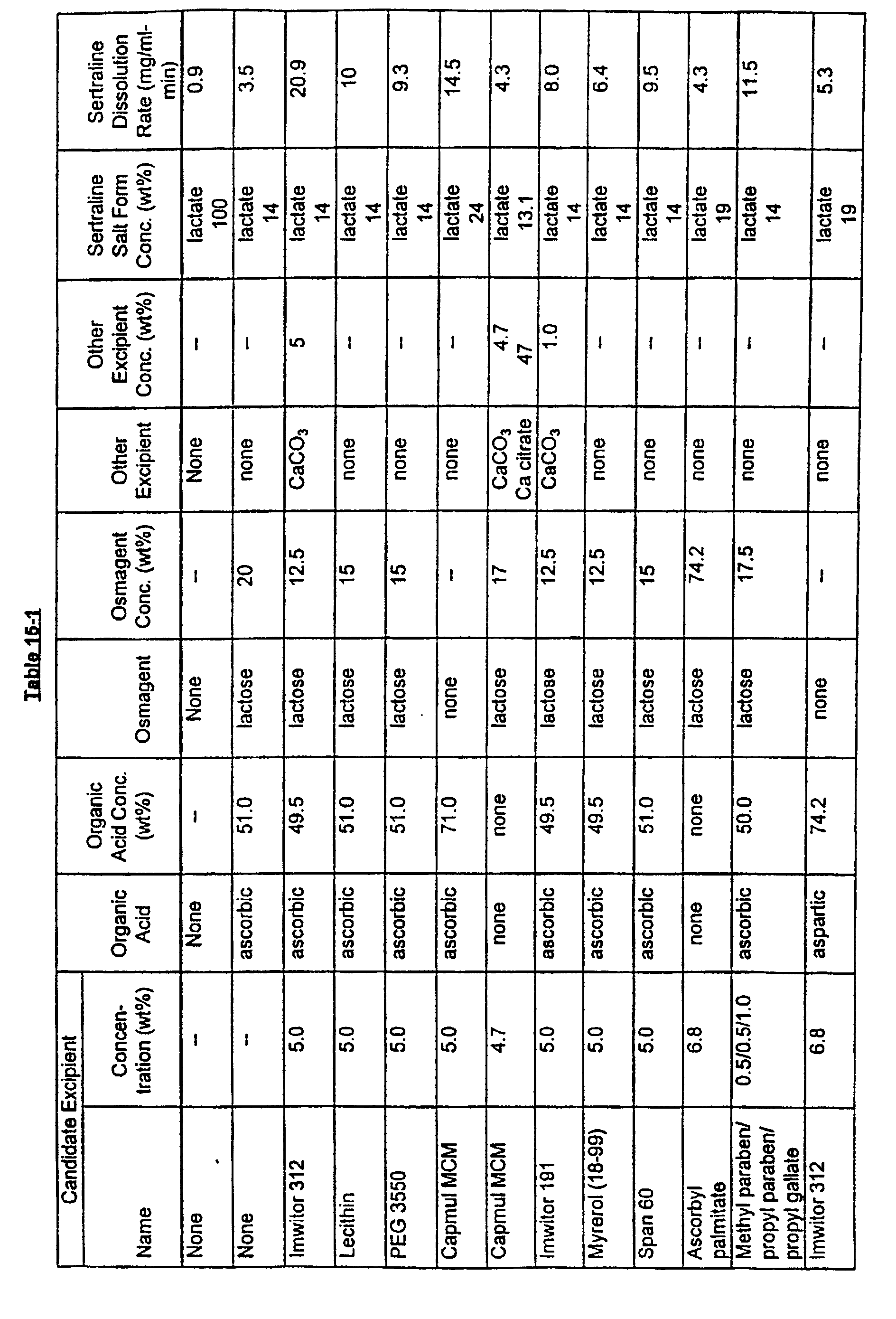

## Claims

1. A spatially delayed-release oral dosage form suitable for oral administration to a mammal, comprising sertraline or a pharmaceutically acceptable salt thereof and a pharmaceutically acceptable carrier, which dosage form, following ingestion by said mammal, releases not more than 10% of the sertraline contained therein into said mammal's stomach, and which effects immediate release of the remaining sertraline contained therein after having passed into said mammal's small intestine.

2. A dosage form as defined in claim 1, which is pH-triggered.

3. A dosage form as defined in claim 2, comprising an immediate-release core coated with a material comprising a polymer that is substantially impermeable to sertraline at the pH of the stomach, but which is permeable to sertraline at the pH of the small intestine.

4. A dosage form as defined in claim 3, in the form of a gelatin capsule coated with a polymer that is substantially impermeable to sertraline at the pH of the stomach, but that is permeable to sertraline at the pH of the small intestine.

5. A dosage form as defined in claim 1, which is enzyme-triggered.

6. A dosage form as defined in claim 5, comprising:
an immediate-release core comprising sertraline and a pharmaceutically acceptable carrier; a membrane surrounding said core, wherein said membrane is fabricated from a microporous hydrophobic material; and a hydrophobic liquid entrained within the pores of said membrane, said hydrophobic liquid being substantially impermeable to water and sertraline, but capable of changing, through enzymatic degradation, so that said membrane becomes substantially permeable to water and sertraline when said dosage form moves into the small intestine.

7. A pH-triggered delayed release dosage form suitable for oral administration to a mammal, comprising (1) an immediate-release core comprising sertraline or a pharmaceutically acceptable salt thereof and a pharmaceutically acceptable carrier, and (2) a pH-sensitive coating surrounding said core, which dosage form, when dissolution tested in vitro, releases not more than 10% of its incorporated sertraline in 2 hours in 750 ml of 0.1 N HCI, and which, following said 2 hours, effects immediate release of its remaining sertraline in a liter of 0.05 M sodium phosphate buffer, pH 6.8, containing 1% polysorbate 80.

8. A dosage form as defined in claim 7, comprising an immediate-release core coated with a polymer that is substantially impermeable to sertraline in said acid but which is permeable to sertraline in said buffer.

9. A dosage form as defined in claim 8, in the form of a gelatin capsule coated with a polymer that is substantially impermeable to sertraline in said acid, but that is permeable to sertraline in said buffer.

10. An enzyme-triggered delayed release dosage form suitable for oral administration to a mammal, comprising (1) an immediate-release core comprising sertraline or a pharmaceutically acceptable salt thereof and a pharmaceutically acceptable carrier, and (2) an enzymatically degradable coating surrounding said core, which dosage form, when dissolution tested in vitro, releases not more than 10% of its incorporated sertraline in 2 hours in 750 ml of 0.1 N HCI, and which, following said 2 hours, effects immediate release of its remaining sertraline in a liter of 0.05 M sodium phosphate buffer, pH 6.8, containing 1% polysorbate 80, in the presence of an enzyme suitable for enzymatically degrading said coating.

11. A dosage form as defined in claim 10, wherein said core is a tablet comprising sertraline and a pharmaceutically acceptable carrier; a membrane surrounding said core, wherein said membrane is fabricated from a microporous hydrophobic material; a hydrophobic liquid entrained within the pores of said membrane, said hydrophobic liquid being substantially impermeable to water and sertraline, but capable of changing, through enzymatic degradation, so that said membrane becomes permeable to water and sertraline in said buffer.

12. A dosage form as defined in claim 11, wherein said core further comprises at least one osmagent.

13. A temporally delayed dosage form suitable for oral administration to a mammal, comprising (1) an immediate release core comprising sertraline or a pharmaceutically acceptable salt thereof and a pharmaceutically acceptable carrier and (2) a coating surrounding said core, which dosage form, following ingestion by said mammal, releases substantially no sertraline during a first period of about 10 minutes, releases not more than 10% of the sertraline contained therein during a second period lasting up to 2 hours following said first period, and then effects immediate release of the remaining sertraline contained therein.

14. A temporally delayed dosage form suitable for administration to a mammal, comprising (1) an immediate-release core comprising sertraline or a pharmaceutically acceptable salt thereof and a pharmaceutically acceptable carrier and (2) a coating surrounding said core, which dosage form, when dissolution tested in vitro in a USP-2 apparatus containing 900 ml of acetic acid/acetate buffer, pH 4.0, which is 0.075 M in NaCI, releases substantially no sertraline during a first period of about 10 minutes, releases not more than 10% of the sertraline contained therein during a second period lasting up to 2 hours following said first period, and which then effects immediate release of the remaining sertraline contained therein following said second period.

15. A delayed-release dosage form suitable for oral administration to a mammal, comprising sertraline or a pharmaceutically acceptable salt thereof and a pharmaceutically acceptable carrier, said dosage form exhibiting, *in vivo,* a sertraline plasma Tₘₐₓ which is shorter than Tₘₐₓ determined after ingestion of an equal amount of sertraline in an immediate release dosage form.

16. A dosage form as defined in claim 15, wherein Tₘₐₓ determined with said delayed release dosage form is at least 0.5 hour shorter than Tₘₐₓ determined with said immediate release dosage form.

17. A dosage form as defined in claim 15, wherein Tₘₐₓ determined with said delayed release dosage form is at least 1hr shorter than Tₘₐₓ determined with said immediate release dosage form.

18. A dosage form as defined in claim 15, which is spatially delayed.

19. A dosage form as defined in claim 15, which is temporally delayed.

20. A dosage form as defined in claim 15, wherein said shortened Tₘₐₓ is determined as the average Tₘₐₓ from dosing at least 12 normal healthy human subjects in a cross-over study in which said immediate release dosage form is an immediate release tablet.

21. A dosage form as defined in claims 3, 5, 8, 10, 13 or 14 wherein said core is a tablet.

22. A dosage form as defined in claims 3, 5, 8, 10, 13 or 14 wherein said core is multiparticulate.

23. A dosage form as defined in claims 1, 10, 13, 14 or 15 wherein said mammal is a human.

24. A dosage form as defined in claims 13 or 14 wherein said core is a tablet coated with a water-soluble or water-disintegrable coating.

25. A dosage form as defined in claims 13 or 14 wherein said core is multiparticulate coated with a water-soluble or water-disintegrable coating.

26. A dosage form as defined in claims 13 or 14 in the form of a gelatin capsule coated with a water-soluble or water-disintegrable coating.

27. A dosage form as defined in claims 3 or 8 wherein said polymer is selected from cellulose acetate phthalate, polyvinylacetate phthalate, hydroxypropylmethylcellulose phthalate, cellulose acetate trimellitate, anionic acrylic copolymers of methacrylic acid and methylmethacrylate, and copolymers comprising acrylic acid and at least one acrylic acid ester.

28. A dosage form as defined in claims 3 or 8 wherein said core is a capsule.

29. The use of sertraline in the preparation of a dosage form in accordance with any one of those described in claims 1, 7, 10, 13, 14 and 15 for the treatment of psychiatric illness, premature ejaculation, chemical dependency, premenstrual dysphoric disorder or obesity.

## Patentansprüche

1. Orale Dosierungsform mit räumlich verzögerter Freisetzung, geeignet zur oralen Verabreichung an einen Säuger, umfassend Sertralin oder ein pharmazeutisch verträgliches Salz davon und einen pharmazeutisch verträglichen Träger, wobei die Dosierungsform nach der Einnahme durch den Säuger nicht mehr als 10% des darin enthaltenen Sertralins in den Magen des Säugers freisetzt und welche unverzügliche Freisetzung des darin verbliebenen Sertralins bewirkt, nachdem sie in den Dünndarm des Säugers gelangt ist.

2. Dosierungsform nach Anspruch 1, die pH-Wert-getriggert ist.

3. Dosierungsform nach Anspruch 2, umfassend einen Kern mit unverzüglicher Freisetzung, beschichtet mit einem Material, umfassend ein Polymer, das bei dem pH-Wert des Magens für Sertralin im Wesentlichen undurchlässig ist, jedoch bei dem pH-Wert des Dünndarms für Sertralin durchlässig ist.

4. Dosierungsform nach Anspruch 3 in Form einer Gelatinekapsel, beschichtet mit einem Polymer, das bei dem pH-Wert des Magens für Sertralin im Wesentlichen undurchlässig ist, jedoch bei dem pH-Wert des Dünndarms für Sertralin durchlässig ist.

5. Dosierungsform nach Anspruch 1, die Enzym-getriggert ist.

6. Dosierungsform nach Anspruch 5, umfassend:
einen Kern mit unverzüglicher Freisetzung, umfassend Sertralin und einen pharmazeutisch verträglichen Träger; eine den Kern umgebende Membran, wobei die Membran aus einem mikroporösen hydrophoben Material hergestellt ist; und eine hydrophobe Flüssigkeit, mitgeführt innerhalb der Poren der Membran, wobei die hydrophobe Flüssigkeit für Wasser und Sertralin im Wesentlichen undurchlässig ist, jedoch infolge eines enzymatischen Abbaus so ausgetauscht werden kann, dass die Membran für Wasser und Sertralin im Wesentlichen durchlässig wird, wenn sich die Dosierungsform in den Dünndarm hinein bewegt.

7. pH-Wert-getriggerte Dosierungsform mit verzögerter Freisetzung, geeignet zur oralen Verabreichung an einen Säuger, umfassend (1) einen Kern mit unverzüglicher Freisetzung, umfassend Sertralin oder ein pharmazeutisch verträgliches Salz davon und einen pharmazeutisch verträglichen Träger, und (2) eine pH-Wert-empfindliche, den Kern umgebende Beschichtung, wobei die Dosierungsform, wenn sie in vitro bezüglich der Auflösung getestet wird, innerhalb von 2 Stunden in 750 ml 0,1 N HCl nicht mehr als 10% ihres enthaltenen Sertralins freisetzt und welche nach den 2 Stunden die unverzügliche Freisetzung ihres verbliebenen Sertralins in einem Liter eines 0,05 M Natriumphosphatpuffers, pH-Wert 6,8, enthaltend 1% Polysorbat 80, bewirkt.

8. Dosierungsform nach Anspruch 7, umfassend einen Kern mit unverzüglicher Freisetzung, beschichtet mit einem Polymer, das in der Säure für Sertralin im Wesentlichen undurchlässig ist, jedoch in dem Puffer für Sertralin durchlässig ist.

9. Dosierungsform nach Anspruch 8 in Form einer Gelatinekapsel, beschichtet mit einem Polymer, das in der Säure für Sertralin im Wesentlichen undurchlässig ist, jedoch in dem Puffer für Sertralin durchlässig ist.

10. Enzym-getriggerte Dosierungsform mit verzögerter Freisetzung, geeignet zur oralen Verabreichung an einen Säuger, umfassend (1) einen Kern mit unverzüglicher Freisetzung, umfassend Sertralin oder ein pharmazeutisch verträgliches Salz davon und einen pharmazeutisch verträglichen Träger, und (2) eine enzymatisch abbaubare, den Kern umgebende Beschichtung, wobei die Dosierungsform, wenn sie in vitro bezüglich der Auflösung getestet wird, innerhalb von 2 Stunden in 750 ml 0,1 N HCl nicht mehr als 10% ihres enthaltenen Sertralins freisetzt und wobei sie nach den 2 Stunden in Gegenwart eines für den enzymatischen Abbau der Beschichtung geeigneten Enzyms die unverzügliche Freisetzung des verbleibenden Sertralins in einem Liter eines 0,05 M Natriumphosphatpuffers, pH-Wert 6,8, enthaltend 1% Polysorbat 80, bewirkt.

11. Dosierungsform nach Anspruch 10, wobei der Kern eine Tablette ist, umfassend Sertralin und einen pharmazeutisch verträglichen Träger; eine den Kern umgebende Membran, wobei die Membran aus einem mikroporösen hydrophoben Material hergestellt ist; eine hydrophobe Flüssigkeit, mitgeführt innerhalb der Poren der Membran, wobei die hydrophobe Flüssigkeit für Wasser und Sertralin im Wesentlichen undurchlässig ist, jedoch infolge des enzymatischen Abbaus ausgetauscht werden kann, sodass die Membran für Wasser und Sertralin in dem Puffer im Wesentlichen durchlässig wird.

12. Dosierungsform nach Anspruch 11, wobei der Kern des weiterhin ein Osmagent umfasst.

13. Dosierungsform mit zeitlich verzögerter Freisetzung, geeignet zur oralen Verabreichung an einen Säuger, umfassend (1) einen Kern mit unverzüglicher Freisetzung, umfassend Sertralin oder ein pharmazeutisch verträgliches Salz davon und einen pharmazeutisch verträglichen Träger, und (2) eine den Kern umgebende Beschichtung, wobei die Dosierungsform nach der Einnahme durch den Säuger während eines ersten Zeitraums von etwa 10 Minuten im Wesentlichen kein Sertralin freisetzt, während eines zweiten 2 Stunden dauernden Zeitraums, der dem ersten Zeitraum folgt, nicht mehr als 10% des darin enthaltenen Sertralins freisetzt und anschließend die unverzügliche Freisetzung des darin verbliebenen Sertralins bewirkt.

14. Dosierungsform mit zeitlich verzögerter Freisetzung, geeignet zur Verabreichung an einen Säuger, umfassend (1) einen Kern mit unverzüglicher Freisetzung, umfassend Sertralin oder ein pharmazeutisch verträgliches Salz davon und einen pharmazeutisch verträglichen Träger, und (2) eine den Kern umgebende Beschichtung, wobei die Dosierungsform, wenn sie bezüglich der Auflösung in vitro in einer USP-2-Vorrichtung, enthaltend 900 ml Essigsäure/Acetatpuffer pH-Wert 4,0, die 0,075 M NaCl enthält, getestet wird, während eines ersten Zeitraums von etwa 10 Minuten im Wesentlichen kein Sertralin freisetzt, während eines zweiten 2 Stunden dauernden Zeitraums, der dem ersten Zeitraum folgt, nicht mehr als 10% des darin enthaltenen Sertralins freisetzt und anschließend nach dem zweiten Zeitraum die unverzügliche Freisetzung des darin enthaltenen, verbliebenen Sertralins bewirkt.

15. Dosierungsform mit verzögerter Freisetzung, geeignet zur oralen Verabreichung an einen Säuger, umfassend Sertralin oder ein pharmazeutisch verträgliches Salz davon und einen pharmazeutisch verträglichen Träger, wobei die Dosierungsform in vivo eine Sertralin-Plasma Tₘₐₓ aufweist, die kürzer als die Tₘₐₓ ist, die nach der Einnahme einer gleichen Menge an Sertralin in einer Dosierungsform mit unverzüglicher Freisetzung bestimmt wird.

16. Dosierungsform nach Anspruch 15, wobei Tₘₐₓ, bestimmt mit der Dosierungsform mit verzögerter Freisetzung, mindestens 0,5 Stunden kürzer ist als Tₘₐₓ, bestimmt mit der Dosierungsform mit unverzüglicher Freisetzung.

17. Dosierungsform nach Anspruch 15, wobei Tₘₐₓ, bestimmt mit der Dosierungsform mit verzögerter Freisetzung, mindestens 1 Stunde kürzer ist als Tₘₐₓ, bestimmt mit der Dosierungsform mit unverzüglicher Freisetzung.

18. Dosierungsform nach Anspruch 15, die räumlich verzögert ist.

19. Dosierungsform nach Anspruch 15, die zeitlich verzögert ist.

20. Dosierungsform nach Anspruch 15, wobei die verkürzte Tₘₐₓ als durchschnittliche Tₘₐₓ von einer Dosierung bei mindestens 12 normalen gesunden Menschen in einer Cross-over-Untersuchung bestimmt wird, in der die Dosierungsform mit unverzüglicher Freisetzung eine Tablette mit unverzüglicher Freisetzung ist.

21. Dosierungsform nach Ansprüchen 3, 5, 8, 10, 13 oder 14, wobei der Kern eine Tablette ist.

22. Dosierungsform nach Ansprüchen 3, 5, 8, 10, 13 oder 14, wobei der Kern aus vielen Teilchen besteht.

23. Dosierungsform nach Ansprüchen 1, 10, 13, 14 oder 15, wobei der Säuger ein Mensch ist.

24. Dosierungsform nach Ansprüchen 13 oder 14, wobei der Kern eine mit einer wasserlöslichen oder in Wasser zerfallenden Beschichtung beschichtete Tablette ist.

25. Dosierungsform nach Ansprüchen 13 oder 14, wobei der Kern aus vielen mit einer wasserlöslichen oder in Wasser zerfallenden Beschichtung beschichteten Teilchen besteht.

26. Dosierungsform nach Ansprüchen 13 oder 14 in Form einer mit einer wasserlöslichen oder in Wasser zerfallenden Beschichtung beschichteten Gelatinekapsel.

27. Dosierungsform nach Ansprüchen 3 oder 8, wobei das Polymer aus Celluloseacetatphthalat, Polyvinylacetatphthalat, Hydroxypropylmethylcellulosephthalat, Celluloseacetattrimellitat, anionischen Acrylsäure-Copolymeren von Methacrylsäure und Methacrylsäuremethylester, und Copolymeren, umfassend Acrylsäure und mindestens einen Acrylsäureester, ausgewählt ist.

28. Dosierungsform nach Ansprüchen 3 oder 8, wobei der Kern eine Kapsel ist.

29. Verwendung von Sertralin bei der Herstellung einer Dosierurigsform nach einer von jenen, beschrieben in den Ansprücheri 1, 7, 10, 13, 14 und 15, zur Behandlung psychiatrischer Erkrankungen, vorzeitiger Ejakulation, Abhängigkeit von chemischen Stoffen, prämenstrueller dysphorischer Störung oder Fettleibigkeit.

## Revendications

1. Forme posologique orale à libération retardée spatialement, convenable pour l'administration orale à un mammifère, comprenant de la sertraline ou un de ses sels pharmaceutiquement acceptables et un support pharmaceutiquement acceptable, forme posologique qui, après ingestion par ledit mammifère, libère une quantité non supérieure à 10 % de la sertraline qui s'y trouve dans l'estomac dudit mammifère, et qui provoque la libération immédiate de la sertraline restante qui s'y trouve après passage dans l'intestin grêle dudit mammifère.

2. Forme posologique suivant la revendication 1, qui est déclenchée par le pH.

3. Forme posologique suivant la revendication 2, comprenant un noyau à libération immédiate enrobé d'une matière comprenant un polymère qui est pratiquement imperméable à la sertraline aupH de l'estomac, mais qui est perméable à la sertraline au pH de l'intestin grêle.

4. Forme posologique suivant la revendication 3, sous forme d'une capsule de gélatine enrobée avec un polymère qui est pratiquement imperméable à la sertraline au pH de l'estomac, mais qui est perméable à la sertraline au pH de l'intestin grêle.

5. Forme posologique suivant la revendication 1, qui est déclenchée par une enzyme.

6. Forme posologique suivant la revendication 5, comprenant :
un noyau à libération immédiate comprenant de la sertraline et un support pharmaceutiquement acceptable ; une membrane entourant ledit noyau, ladite membrane étant produite à partir d'une matière hydrophobe microporeuse ; et un liquide hydrophobe entraîné dans les pores de ladite membrane, ledit liquide hydrophobe étant pratiquement imperméable à l'eau et à la sertraline, mais étant apte à une modification, par dégradation enzymatique, de telle sorte que ladite membrane devienne substantiellement perméable à l'eau et à la sertraline lorsque ladite forme posologique migre dans l'intestin grêle.

7. Forme posologique à libération retardée déclenchée par le pH, apte à l'administration orale à un mammifère, comprenant (1) un noyau à libération immédiate comprenant de la sertraline ou un de ses sels pharmaceutiquement acceptables et un support pharmaceutiquement acceptable, et (2) un enrobage sensible au pH entourant ledit noyau, forme posologique qui, lors de la dissolution testée in vitro, libère une quantité non supérieure à 10 % de la sertraline qu'elle renferme en un temps de 2 heures dans 750 ml de HCl 0,1 N, et qui, après ladite période de temps de 2 heures, provoque la libération immédiate de sa sertraline restante dans un litre de tampon au phosphate de sodium 0,05 M, à pH 6,8, contenant 1 % de polysorbate 80.

8. Forme posologique suivant la revendication 7, comprenant un noyau à libération immédiate enrobé d'un polymère qui est pratiquement imperméable à la sertraline dans ledit acide mais qui est perméable à la sertraline dans ledit tampon.

9. Forme posologique répondant à la définition suivant la revendication 8, sous forme d'une capsule de gélatine enrobée d'un polymère qui est pratiquement imperméable à la sertraline dans ledit acide, mais qui est perméable à la sertraline dans ledit tampon.

10. Forme posologique à libération retardée déclenchée par une enzyme, apte à l'administration orale à un mammifère, comprenant (1) un noyau à libération immédiate comprenant de la sertraline ou un de ses sels pharmaceutiquement acceptables et un support pharmaceutiquement acceptable, et (2) un enrobage enzymatiquement dégradable entourant ledit noyau, forme posologique qui, lors de la dissolution testée in vitro, libère une quantité non supérieure à 10 % de la sertraline qu'elle renferme en un temps de 2 heures dans 750 ml de HCl 0,1 N, et qui, après ladite période de temps de 2 heures, provoque la libération immédiate de sa sertraline restante dans un litre de tampon au phosphate de sodium 0,05 M, à pH 6,8, contenant 1 % de polysorbate 80, en présence d'une enzyme convenable pour la dégradation enzymatique dudit enrobage.

11. Forme posologique suivant la revendication 10, dans laquelle ledit noyau est un comprimé comprenant de la sertraline et un support pharmaceutiquement acceptable ; une membrane entourant ledit noyau, ladite membrane étant produite à partir d'une matière hydrophobe microporeuse ; un liquide hydrophobe entraîné dans les pores de ladite membrane, ledit liquide hydrophobe étant pratiquement imperméable à l'eau et à la sertraline, mais étant apte à la modification, par dégradation enzymatique, de telle sorte que ladite membrane devienne perméable à l'eau et à la sertraline dans ledit tampon.

12. Forme posologique suivant la revendication 11, dans laquelle ledit noyau comprend en outre au moins un agent osmotique.

13. Forme posologique à libération retardée dans le temps, apte à l'administration orale à un mammifère, comprenant (1) un noyau à libération immédiate comprenant de la sertraline ou un de ses sels pharmaceutiquement acceptables et un support pharmaceutiquement acceptable, et (2) un enrobage entourant ledit noyau, forme posologique qui, après ingestion par ledit mammifère, ne libère pratiquement pas de sertraline pendant une première période de temps d'environ 10 minutes, libère une quantité non supérieure à 10 % de la sertraline qu'elle renferme au cours d'une seconde période de temps ayant une durée allant jusqu'à 2 heures après ladite première période de temps, puis provoque la libération immédiate de la sertraline restante qu'elle renferme.

14. Forme posologique à libération retardée dans le temps, apte à l'administration à un mammifère, comprenant (1) un noyau à libération immédiate comprenant de la sertraline ou un de ses sels pharmaceutiquement acceptables et un support pharmaceutiquement acceptable et (2) un enrobage entourant ledit noyau, forme posologique qui, lors de la dissolution testée in vitro dans un appareil USP-2 contenant 900 ml de tampon à l'acide acétique/acétate à pH 4,0 ayant une concentration en NaCl de 0,075 M, ne libère pratiquement pas de sertraline au cours d'une première période de temps d'environ 10 minutes, libère une quantité non supérieure à 10 % de la sertraline qu'elle renferme au cours d'une seconde période de temps d'une durée allant jusqu'à 2 heures après ladite première période de temps, et provoque ensuite la libération immédiate de la sertraline restante qu'elle renferme après ladite seconde période de temps.

15. Forme posologique à libération retardée, apte à l'administration orale à un mammifère, comprenant de la sertraline ou un de ses sels pharmaceutiquement acceptables et un support pharmaceutiquement acceptable, ladite forme posologique présentant, *in vivo*, une valeur de Tₘₐₓ plasmatique de sertraline qui est inférieure à la valeur de Tₘₐₓ déterminée après ingestion d'une quantité égale de sertraline dans une forme posologique à libération immédiate.

16. Forme posologique suivant la revendication 15, dans laquelle la valeur de Tₘₐₓ déterminée avec ladite forme posologique à libération retardée est inférieure d'au moins 0,5 heure à la valeur de Tₘₐₓ déterminée avec ladite forme posologique à libération immédiate.

17. Forme posologique suivant la revendication 15, dans laquelle la valeur de Tₘₐₓ déterminée avec ladite forme posologique à libération retardée est inférieure d'au moins 1 heure à la valeur de Tₘₐₓ déterminée avec ladite forme posologique à libération immédiate.

18. Forme posologique suivant la revendication 15, qui est retardée spatialement.

19. Forme posologique suivant la revendication 15, qui est retardée dans le temps.

20. Forme posologique suivant la revendication 15, dans laquelle ladite valeur de Tₘₐₓ plus brève est déterminée par la valeur de Tₘₐₓ moyenne par administration à au moins 12 sujets humains sains normaux dans une étude croisée dans laquelle ladite forme posologique à libération immédiate est un comprimé à libération immédiate.

21. Forme posologique suivant la revendication 3, 5, 8, 10, 13 ou 14, dans laquelle ledit noyau est un comprimé.

22. Forme posologique suivant la revendication 3, 5, 8, 10, 13 ou 14, dans laquelle ledit noyau est constitué de particules multiples.

23. Forme posologique suivant la revendication 1, 10, 13, 14 ou 15, dans laquelle ledit mammifère est un homme.

24. Forme posologique suivant la revendication 13 ou 14, dans laquelle ledit noyau est un comprimé enrobé d'un enrobage hydrosoluble ou apte à la dissociation dans l'eau.

25. Forme posologique suivant la revendication 13 ou 14, dans laquelle ledit noyau est un noyau multiparticules enrobé d'un enrobage hydrosoluble ou apte à la dissociation dans l'eau.

26. Forme posologique suivant la revendication 13 ou 14, sous forme d'une capsule de gélatine enrobée d'un enrobage hydrosoluble ou apte à la dissociation dans l'eau.

27. Forme posologique suivant la revendication 3 ou 8, dans laquelle ledit polymère est choisi entre l'acétophtalate de cellulose, l'acétophtalate de polyvinyle, le phtalate d'hydroxypropylméthylcellulose, l'acétotrimellitate de cellulose, des copolymères acryliques anioniques d'acide méthacrylique et de méthacrylate de méthyle, et des copolymères comprenant de l'acide acrylique et au moins un ester d'acide acrylique.

28. Forme posologique suivant la revendication 3 ou 8, dans laquelle ledit noyau est une capsule.

29. Utilisation de sertraline dans la préparation d'une forme posologique suivant l'une quelconque des formes posologiques décrites dans les revendications 1, 7, 10, 13, 14 et 15 pour le traitement d'une maladie psychiatrique, de l'éjaculation prématurée, d'une dépendance chimique, d'un trouble dysphorique prémenstruel ou de l'obésité.
